# EUROPEAN PATENT APPLICATION

(11) **EP 3 537 153 A1**
(43) Date of publication of application: **11.09.2019**
(21) Application number: 18159973.9
(22) Date of filing: 05.03.2018
(51) Int. Cl.: G01N 33/543, G01N 27/333, G01N 33/84

(54) **MEANS FOR THE QUANTITATIVE DETERMINATION OF AN ANALYTE CONCENTRATION IN A PATIENT'S URINE SAMPLE**

(71) Applicant: Fuhrmann, Gerda Laura, 6094 Axams (AT); Kilickiran, Pinar, 6020 Innsbruck (AT)
(72) Inventor: Fuhrmann, Gerda Laura, 6094 Axams (AT); Kilickiran, Pinar, 6020 Innsbruck (AT)
(74) Representative: Engelhard, Markus

(57) **Abstract**

The present invention relates to a single-use monoanalytical test-strip for the quantitative determination of an analyte in a patient's urine sample, and to a non-invasive point-of-care (POC) device for detecting deficiency and/or overload of an analyte in a patient's body. Furthermore, the present invention relates to a method for quantitatively determining the concentration of an analyte in a patient's urine sample and to a method of detecting deficiency and/or overload of an analyte in a patient's body. The test-strip comprises a substrate which either is electrically insulating or which has an electrically insulating layer applied thereon,- an electrode assembly applied on said substrate or on said electrically insulating layer, if present; and said electrode assembly comprises at least- one analyte-selective working electrode;- one reference electrode for said analyte-selective working electrode and an interface for electrically connecting said electrode assembly to a read out-meter device. Analytes are anions selected from halides, phopshates and sulfates or cations of alkali metals, of alkaline earth metals or of other metals.

## Description

### FIELD OF THE INVENTION

The present invention relates to a single-use monoanalytical test-strip for the quantitative determination of an analyte in a patient's urine sample, and to a non-invasive point-of-care (POC) device for detecting deficiency and/or overload of an analyte in a patient's body. Furthermore, the present invention relates to a method for quantitatively determining the concentration of an analyte in a patient's urine sample and to a method of detecting deficiency and/or overload of an analyte in a patient's body.

### BACKGROUND OF THE INVENTION

The body contains a large variety of analytes which are charged compounds that control a variety of important physiological functions. Each of these charged compounds, e.g. electrolytes, trace elements and minerals in the human or animal body serves a specific role. Their well-balanced distribution is very important for body functions such as hydration, extra- and intracellular pH, metabolism, nerve impulses and muscle contraction/relaxation. Essential analytes, which are electrolytic ions with a positive charge include sodium (Na⁺), potassium (K⁺), calcium (Ca²⁺), magnesium (Mg²⁺), while some examples with negative charge include chloride (Cl⁻) and phosphate (in form of HPO₄²⁻, H₂PO₄⁻ and PO₄³⁻). Further examples of medically relevant analytes are lithium (Li⁺), lead (Pb²⁺), copper (Cu2⁺), zinc (Zn²⁺), iodide (I⁻, as index for iodine body status), sulfate (in form of HSO₄⁻ and SO₄²⁻), creatinium (Crea⁺), ammonium (NH₄⁺), and hydrogen ion (H⁺), which are not electrolytes in the classic sense, but which affect health of a patient and thus their levels should be monitored ([1], [2]).

Disorders including deficiency and/or overload of such analytes, e.g. electrolytes, trace elements, and minerals, in the body of a patient can be detrimental to the patient's health. Serious disorders may arise from inadequate uptake and/or disturbed excretion of these analytes, which may occur during various acute and chronic disease states, e.g. malfunctioning of the kidney, gastrointestinal diseases, infections that produce severe and continual diarrhea or vomiting, and due to drugs that cause loss of electrolytes in the urine (diuretics).

For example, sodium is essential for the maintenance of the normal distribution of fluid between the intra- and extracellular space. It thus regulates blood volume, blood pressure, transport of nutrients and cellular waste products, tissue growth, neurotransmission and multiple other functions. Assessment of the body sodium status is mostly performed to evaluate the state of hydration of the body ([3], [4]). Furthermore, it is useful for monitoring salt intake or salt losses in patients known to be at high risk of sodium disorders including overload and deficiency ([5]).
Sodium overload (NaO) results mainly from the oversalted food commonly offered by the modern food industry. It is well known as a major modifiable risk factor worldwide for arterial hypertension and its sequelae such as cardiac insufficiency and stroke ([6], [7]). The most effective way to solve this problem is to reduce salt/sodium intake. In this respect, a method for monitoring sodium intake and body status is essential.
On the other hand, sodium deficiency (NaD) is an often underestimated and neglected disorder that results from either enhanced loss or insufficient intake of salt [8]. Increased loss may occur in patients with diarrhea, gastrointestinal stoma, cystic fibrosis or during phases of intense physical exercise with enhanced sweating. Low sodium intake may result from eating disorders e.g. anorexia nervosa in adolescents or chronic denial of food by the elderly. In many cases, when patients suffer from proportionate losses of body Na and fluid, a hidden form (normonatremic) NaD may occur, which can hardly be detected in blood ([9], [10]). NaD may cause common nonspecific symptoms such as sleeping disorders, fatigue, headache, confusion, low appetite or growth failure, but may also lead to severe complications such as muscle cramps, epilepsy, encephalopathy, coma and even death ([11], [12]). A test for the timely identification of NaD patients and monitoring during supplementation is therefore urgently needed.

As a further example, potassium is the major intracellular cation and its balance is critical for the normal functioning of muscles, heart, and nerves. It is also important for normal transmission of electrical signals throughout the nervous system. Potassium imbalance may be caused by inadequate intake, altered excretion, or transcellular shifts ([13]). Assessment of body potassium status is mostly performed to evaluate potential causes of cardiac arrhythmia, muscular weakness, hepatic encephalopathy and renal failure. A regular monitoring is needed especially in case of diabetic ketoacidosis.
Potassium overload (KO) may occur associated with kidney disease, severe dehydration, severe acidosis, including diabetic ketoacidosis, and certain medications, including some blood pressure medications. KO can be asymptomatic, meaning that it causes only vague symptoms including nausea, fatigue, muscle weakness, or tingling sensations. However, if left undiagnosed and untreated, the condition can be fatal leading to cardiac arrest and death. Patients with chronic KO are counseled to reduce dietary potassium, for which a close monitoring is needed.
Potassium deficiency (KD) is often caused by severe vomiting or diarrhea, dehydration, certain medications, including laxatives, diuretics, and corticosteroids or as as a result of eating disorders. Minor cases may have lesser symptoms, like muscle weakness and cramping, but severe cases can be deadly and must be treated immediately. Usually, for treatment, oral potassium supplementation is recommended; however, careful monitoring during treatment is essential because supplemental potassium is also a common cause of KO, for example in hospitalized patients.

As a further example, calcium is necessary for muscle contraction, enzyme activity, and blood coagulation. In addition, calcium helps to stabilize cell membranes and is essential for the release of neurotransmitters from neurons and of hormones from endocrine glands. Sufficient bioavailable calcium is essential for bone health ([14], [15]). Assessment of body calcium status is mostly performed for the evaluation of bone diseases, including osteoporosis and osteomalacia. Osteoporosis is a very common bone disorder that may affect females and males of all ages. During osteoporosis treatment, monitoring of calcium status is necessary, as the milk-alkali syndrome may emerge when supplementation is too high. Further, assessment of calcium levels, in particular in urine, is performed for the evaluation of kidney stone risk. Calcium disorders may for long times show little symptomatology, thus, the status should be tested regularly. Calcium deficiency (CaD) may be a sign for inadequate dietary calcium intake and/or a high oxalate diet as well as malabsorption disorders, e.g. vitamin D deficiency or hypoparathyroidism. Calcium overload (CaO) may indicate a high calcium diet, but also for example kidney disease, hyperparatyroidism, sarcoidosis or bone metastases.

As a further example, magnesium is the second most abundant intracellular cation and it is a cofactor in many enzymatic reactions, particularly those involving the formation or utilisation of ATP. Magnesium affects many cellular functions, including transport of potassium and calcium ions, and modulates signal transduction, energy metabolism and cell proliferation ([16]). Assessment of magnesium status is mostly performed for determining whether nutritional magnesium loads are adequate and in combination with a calcium test for assessing kidney stone risk ([15]). Magnesium deficiency (MgD), mainly results from low magnesium in the diet or some other disease such as celiac disease, other malabsorption disorders, dysbiosis, vitamin D deficiency, pancreatic insufficiency. MgD may increase the incidence of oxalate crystal formation in the tissues and kidney stones. As MgD worsens, numbness, tingling, muscle contractions and cramps, seizures, anxiety, depression, attention deficit, abnormal heart rhythms, and coronary spasms can occur. Magnesium overload (MgO) mainly results from high amounts of magnesium in the diet. Symptoms can include diarrhea, hypotension, nausea, vomiting, facial flushing, retention of urine, ileus, depression, lethargy, muscle weakness, breathing difficulty, extreme hypotension, irregular heartbeat, and cardiac arrest.

A further exemplary analyte is chloride which is the major anion in the extracellular water space; its physiological significance is maintaining proper body water distribution, osmotic pressure, and normal anion-cation balance in the extracellular fluid compartment. Usually, for clinical relevance, a chloride test is performed in combination with additional supplemental tests, such as potassium or sodium status test ([17]). Assessment of body chloride status is mostly performed for evaluating hydration status and acid-base homeostasis of the body.

A further exemplary analyte is phosphate which is present in the body in three ionic forms: H₂PO₄⁻, HPO₄²⁻, and PO₄³⁻. Bone and teeth bind up 85 percent of the body's phosphate as calcium-phosphate salts. Phosphate is found in phospholipids, such as those that make up the cell membrane, and in ATP, nucleotides, and buffers ([18]). Assessment of body phosphate status is mostly performed for evaluation of hypo- and hyper-phosphataemic states and patients with nephrolithiasis. Further, phosphate levels are detected for diagnosis and management of a variety of disorders including bone, parathyroid and renal disease.

A further exemplary analyte is iodine (I) which is significant to health and is a component of hormones produced by the thyroid. Iodine must be obtained through dietary sources, because the body does not produce it ([19]). Assessment of body iodine status is mostly performed for determining whether nutritional iodine loads are adequate, and for monitoring iodine level in patients with various thyroid diseases and in pregnant women. Urinary excretion of iodide is the most valid index of iodine intake and in general, the assessment is done using urine. Iodine deficiency (ID) is a leading cause of thyroid disorders and it can manifest as hypothyroidism or impaired mental function. Insufficient levels of iodine increase the risk of thyroid cancer and may be associated with risk of breast, ovarian, endometrial, stomach, and esophageal cancer. ID during pregnancy is associated with high risk of mental retardation and cretinism of newborns. Iodine supplements are effective in preventing and treating iodine deficiency and in achieving whole-body iodine sufficiency. However, in order to avoid adverse effects such as hyperthyroidism and endemic goiter, iodine supplementation requires proper monitoring.

A further exemplary analyte is zinc (Zn) which is an essential element involved in a variety of enzyme systems and processes including wound healing, immune function and fetal development. Zinc deficiency (ZnD) may occur in patients with gastrointestinal disorders like celiac or Crohn's disease, Wilson's disease, diabetes, chronic liver and kidney disease ([20]). Assessment of body zinc status is mostly performed to evaluate suspected nutritional inadequacy, cases of diabetes or delayed wound healing, growth retardation or to monitor therapies like oral zinc therapy used in Wilson's disease.

A further exemplary analyte is copper (Cu) which is part of several proteins and an essential element for many biological processes. Copper deficiency (CuD) is seen in malnutrition, hypoproteinemias, malabsorption, nephrotic syndrome, and patients with Menkes disease. Copper can be toxic when present in excess often causing severe liver damage ([21]). Copper overload (CuO) occurs mainly in people with Wilson's disease. The main sources of copper for humans are food, drinking water and copper-containing supplements. In general, copper content in the diet varies widely because foodstuffs differ greatly in their natural copper content. Factors such as season, soil quality, geography, water source and use of fertilizers influence the final copper content in food. Assessment of body copper status is mostly performed to determine suspected nutritional inadequacy, to monitor therapies of Wilson's disease, and to examine obstructive liver disease.

A further exemplary analyte is lithium (Li) which is used in the treatment of mania and in preventing the recurrence of both manic and depressive symptomatology. The therapeutic range for lithium is established; however, response and side effects, and therefore the dosage, are personalized ([22]). Side effects and toxicity from lithium salts lead to ataxia, slurred speech, confusion and increased risk of cardiovascular disease. Assessment of body lithium status is thus performed to determine the therapeutic level when starting medication, and for monitoring therapy in order to prevent intoxication of patients.

A further exemplary analyte is lead (Pb) which is a toxic metal that may cause life-threatening poisoning. Lead compounds have numerous commercial and industrial applications, such as in paints, plastics, storage batteries, bearing alloys, insecticides, and ceramics. A common source of lead exposure among children is due to contacting their mouth with Pb-containing objects, specifically objects with paint containing lead ([23]). Lead poisoning through chronic exposure is characterized by gastrointestinal disturbance, anemia, insomnia, weight loss, muscle weakness and paralysis, neuropathy and on long-term or large quantities may produce death. The assessment of body lead status is mostly done for detecting clinically significant lead exposure, for routine health screening, in particular, if toxicity is suspected, and for monitoring the indicated treatment that is mostly based on a lead chelation therapy.

A further exemplary analyte is creatinine which is a waste product of muscle metabolism in the body, and which is excreted through urine without reabsorption at a relatively constant rate through glomerular filtration. Within an individual, the excretion is relatively stable, unless a high protein diet is ingested ([24]). Assessment of body creatinine status is critically important and it has several properties. Creatinine level in blood is a marker of the glomerular filtration rate. Urinary creatinine, in conjunction with serum creatinine, is used to calculate the creatinine clearance, a measure of renal function. Urinary creatinine can also be used as normalization factor for other analyte concentrations ([25]).

A further exemplary analyte is sulfate (inorganic) which is primarily a biodegradation product of the amino acids methionine and cysteine that are mainly contained in meat and fish. The main excretion route of sulfate is through urine. Sulfate is the major anion in urine that has significant affinity to cations such as calcium, and a modulating role for their availability to react with other anions. Thus, the level of sulfate in urine is an important factor for urinary supersaturation for various crystals and stones, such as calcium oxalate and hydroxyapatite ([26]). Assessment of sulfate status in urine is mostly performed to provide information of nutritional protein intake, for calculating the urinary supersaturation of various crystals or stones, as an index for protein-induced calciuria.

A further exemplary analyte is ammonium which is mainly a waste product of proteins. The kidney regulates acid excretion and systemic acid base balance by changing the ammonium concentrations in urine. The assessment of ammonium status is performed to provide understanding of the cause of an acid-base disturbance, for the diagnosis and treatment of kidney stones patients, and timely identification of metabolic acidosis ([17]).

A further exemplary analyte is hydrogen ion which reflects the pH level (acidity or alkalinity) of a sample. The assessment of the pH of urine is mostly performed to detect kidney stones, to monitor treatment and diet adjustment, to provide information on possible acidosis, dehydration, diabetic ketoacidosis, effects of gastric suctioning, kidney failure, and urinary tract infection ([1], [2]).

Ion-selective electrodes (ISEs) are an efficient and versatile tool for charged compounds, such as electrolytes, and ion detection ([27]). However, portability and applicability for field applications are limited by the need of a bulky reference electrode, conditioning step, and the high cost.

With the advent of electrochemical device miniaturization enabled by advanced technologies, such as screen printing or inkjet, and maturation of mobile technology, a new era of POC-testing has emerged ([28], [29]) potentially allowing the quantitative measurement of a variety of analytes as has been demonstrated for glucose in patients with diabetes mellitus.

WO 2015/133706 discloses a portable digital measurement device and method for multiplexed measuring sodium and potassium in urine.

WO 2016/156941A1 discloses an ion-selective membrane for use in an electrochemical biosensor for analysing electrolytes in blood samples.

Many of the present routines for assessing an analyte in a body, e.g. human body sodium status, are measurements of the analyte concentration in blood. The major analytical methods applied are indirect potentiometry ("indirect" = sample dilution) by means of a classical ion-selective electrode (ISE), spectrophotometry, atomic absorption spectroscopy (AAS) or inductively coupled plasma-mass spectrometry (ICP-MS). For these methods, special instrumentation, often pre-analytical sample preparation and skilled personnel are required. The measurements are performed in centralized clinical laboratories were the samples have to be sent. The time to result may take many hours and even days, hampering timely therapeutic decision. Furthermore, the sampling of the blood is invasive, needs to be done by trained medical staff, usually in a physician's office or in a hospital, and is thus not suited for point-of-care (PoC) diagnostics.

A less-invasive, less-expensive, and easy-to-use means to identify and monitor disorders of an analyte in a body is highly desirable.

Measuring the urinary concentration of an analyte is an easy-to-use method for quantifying intake, as well as an effective way of estimating and monitoring body status of an analyte, since urine is an ideal specimen; it can be collected non-invasively, is usually available in plentiful amounts and poses minimal risks.

Accordingly, it was an object of the present invention to provide for means to measure an analyte concentration in a patient's urine sample in a straightforward manner and to provide such means which can be easily handled. It was furthermore an object of the present invention to provide for a methodology for determining the concentration of an analyte in a patient's urine sample for determining deficiency and/or overload of an analyte that is time-efficient and can be practiced at the point-of-care (POC). It was furthermore an object of the present invention to provide for a method for detecting deficiency and/or overload of an analyte in a patient's body which can be practiced even by untrained persons, such as the patient him/herself. It was furthermore an object of the present invention to provide for means and a methodology for quantitatively determining a concentration of an analyte in a patient's urine sample and/or to detect deficiency and/or overload of an analyte in a patient's body that is not time consuming and that can be performed as a routine operation.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a single-use monoanalytical test-strip for the quantitative determination of an analyte in a patient's urine sample, wherein said analyte is selected from cations of alkali metals, preferably selected from Na⁺, K⁺, and Li⁺, alkaline earth metals, preferably selected from Ca²⁺ and Mg²⁺, other metals, which are non-alkali metals and non-alkaline earth metals, preferably selected from Pb²⁺, Zn²⁺, and Cu²⁺, anions selected from halides, in particular Cl⁻ and I⁻, phosphates, and sulfates, and non-metal cations, preferably selected from NH₄⁺, H⁺, and creatinine⁺,
said test-strip comprising:
- a substrate which either is electrically insulating or which has an electrically insulating layer applied thereon,
- an electrode assembly applied on said substrate or on said electrically insulating layer, if present; said electrode assembly comprising at least
   - one analyte-selective working electrode;
   - one reference electrode for said analyte-selective working electrode;
   - optionally; a second analyte-selective working electrode for control measurement, and/or one or two neutral electrodes for measuring and eliminating interferences,
- an interface for electrically connecting said electrode assembly to a read out-meter device, optionally via a test-strip holder unit.

In one embodiment, such determination occurs by potentiometric measurement(s).

In one embodiment, said analyte-selective working electrode, said reference electrode, and said second analyte-selective electrode and/or neutral electrode(s), if present, have been applied on said substrate or on said electrically insulating layer, if present, by a suitable deposition technique, such as printing, sputtering, evaporating, electro-less plating, affixation, gluing or lithography, preferably screen printing or ink-jet printing, thus forming an electrode assembly on said substrate or on said electrically insulating layer, and wherein said analyte-selective working electrode comprises an analyte-selective membrane, and wherein said neutral electrode(s) comprise(s) a membrane that is not analyte-selective.

In one embodiment, said substrate is made of a material selected from plastic, ceramic, alumina, paper, cardboard, rubber, textile, carbon-based polymers, such as polypropylene, fluoropolymers, such as Teflon, silicon-based substrates, such as glass, quartz, silicon nitride, silicon oxide, silicon based polymers such as polydimethoxysiloxane, semiconducting materials such as elemental silicon, dielectric materials, preferably selected from organic dielectric materials such as polyimide, polycarbonate, polyvinyl chloride, polystyrene, polyethylene, polypropylene, polyester, polyethylene terephthalate, polyurethane, polyvinylidene fluoride, inorganic dielectric materials such as silicium dioxide, and wherein said electrically insulating layer, if present, is made of a dielectric material, wherein, if said electrically insulating layer is present on said substrate, said electrode assembly is located on said electrically insulating layer.

In one embodiment, said analyte-selective working electrode comprises an analyte-selective membrane that comprises an analyte-selective carrier in a polymer matrix.

In one embodiment, said electrode assembly further comprises one or two neutral electrodes for measuring and eliminating interferences, wherein said neutral electrode(s) comprise(s) a membrane comprising a polymeric matrix without any analyte-selective carrier.

In one embodiment, said electrode assembly further comprises a second analyte-selective working electrode for control measurement.

In one embodiment, each of said electrodes in said electrode assembly has an electrical lead, respectively, wherein said electrical lead connects said electrode with said interface for electrically connecting said electrode assembly to a readout-meter device.

In one embodiment, said reference electrode has a surface larger than the surface of said working electrode.

In one embodiment, said test-strip is a dipstick test-strip which is contacted with a sample by dipping.

In one embodiment, the working electrodes may be made of any conductive material, such as carbon, gold, palladium, silver, platinum, titanium, chromium, iridium, tin, their oxides or derivatives and combinations thereof such as fluorine doped tin oxide (FTO) or indium tin oxide (ITO). In one embodiment, said electrodes have been applied on said substrate or on said electrically insulating layer, if present, by a suitable deposition technique, such as printing, sputtering, evaporating, electro-less plating, affixation, gluing or lithography, preferably screen printing or ink-jet printing. The electrodes may be individually deposited or together.

In one embodiment, the reference electrode(s) preferably is a Ag/AgCl system, but other suitable reference material(s) providing a controlled potential in biological fluids are also possible and envisaged.

In one embodiment, said reference electrode is made from the same material as the working electrodes and has a surface, which surface is coated with a stabilizing layer such as a conductive polymer e.g. polyaniline, poly(3,4-ethylenedioxythiophene), poly(3-octylthiophene), polypyrrole, or a polymeric material, e.g. polyurethane, polyvinylbutyral, polyvinylchloride, comprising Ag/AgCl/Cl⁻ system for maintaining constant potential of the electrode.

In one embodiment, for a higher potentiometric stability, the reference electrode may be in addition coated with a stabilizing layer consisting polymeric material including lipophilic salts, such as an anion and cation exchange materials (e.g. different tetraalkylammonium(s) and tetraphenylborates). These components of high lipophilicity exclude or minimize ion exchange with the sample solution and confer constant potential of reference electrode.

In a further aspect, the present invention relates to a non-invasive point-of-care (POC) device for detecting and/or determining deficiency and/or overload of an analyte in a patient's body, said POC device comprising:
a readout-meter device for the quantitative and selective measurement of the concentration of an analyte in a urine sample, said readout-meter device comprising
   - a multichannel amplifier, preferably having high input resistance, for amplifying electrical signal(s) transmitted from a single-use test-strip
   - a controller including an analog/digital converter and a storage memory, for converting electrical signals received from a single-use test-strip into analyte concentration measurement(s)
   - a measurement mechanism to start and, preferably, conduct a measurement of an analyte concentration
   - an output device for indicating concentration measurements to a user, preferably a display
   - a receiving module for receiving an interface of a single-use test-strip or of a test-strip holder unit,
   - optionally, a communication unit, and
   - a power supply; and
optionally, a test-strip holder unit for receiving an interface of a single-use test-strip and having an interface for connecting to said receiving module and for establishing electrical contact between said readout-meter device and an electrode assembly of said single-use test-strip, thus allowing the detection and transmission of electrical signal(s) from said single-use test-strip to said readout-meter device, wherein said test-strip holder unit has electrical connectors for separately contacting each electrode via said interface of said test-strip.

In one embodiment, said POC device further comprises a release mechanism to release said test-strip from said receiving module of the readout-meter device or from said test-strip holder unit.

In one embodiment, said POC device mandatorily comprises a test-strip holder unit.

In one embodiment, said measurement mechanism is selected from a measure button, such as a push button, a press button, a touch button, or a digital control button, a lever, a second pressure point of a button of the POC device or pressing a button of the POC device for a second time, and a voice-controlled means to initiate the measurement.

In one embodiment, said release mechanism is selected from a button, such as a push button, a press button, a touch button, or a digital control button, a lever, a second pressure point of a button of the POC device or pressing a button of the POC device for a second time, and a voice-controlled release mechanism.

In one embodiment, said release mechanism is part of the readout-meter device, or said release mechanism is part of the test-strip holder unit.

In one embodiment, said device further comprises a user-interface for operating said device, and/or a memory for storing a plurality of analyte concentration measurements, and/or a communication unit, preferably a USB and/or wireless communication unit, for transferring and/or exchanging data with an external computer or external network.

In one embodiment, said device comprises both a port for USB connection and wireless connection.

In one embodiment, said POC device comprises a test-strip holder unit comprising a main body and at least two terminal interfaces, one for receiving an interface of said test-strip and the other for contacting said receiving module of said readout-meter device, and electrical leads for electrically connecting said digital readout-meter device with said single-use test-strip.

In one embodiment, said device further comprises a single-use test-strip inserted into said receiving module of said readout-meter device or said test-strip holder unit by way of said interface of said single-use test-strip, thus establishing electrical contact between said electrode assembly of said test-strip and said readout-meter device, wherein, preferably, said single-use test-strip is the single-use monoanalytical test-strip as defined further above and below.

In one embodiment, said measurement is as defined herein. Preferably, such measurement is a potentiometric measurement.

In a further aspect, the present invention relates to a method for quantitatively determining the concentration of an analyte in a patient's urine sample, comprising the steps:
a) providing a urine sample
b) connecting a single-use test-strip comprising an electrode assembly, wherein said electrode assembly comprises at least
   - one analyte-selective working electrode;
   - one reference electrode for said analyte-selective working electrode;
   - optionally; a second analyte-selective working electrode for control measurement, and/or one or two neutral electrodes for measuring and eliminating interferences,
   to a readout-meter device of a point-of-care (POC) device as defined in another embodiment of the present invention, to assemble a point-of-care (POC) device as defined in another embodiment of the invention, wherein said single-use test-strip is inserted into said receiving module of said readout-meter device or into said test-strip holder unit contacted to said readout meter device, thus establishing electrical contact between said electrode assembly of said test-strip and said readout-meter device,
c) contacting said test-strip with said urine sample, and allowing said electrode assembly of said test-strip to be wetted by and come into contact with said urine sample, optionally withdrawing the urine-wetted test-strip from said urine sample,
   wherein said connecting of said test-strip to said readout-meter device of said POC device, optionally via said test-strip holder unit, of step b) occurs either before or after step c),
d) measuring the concentration of an analyte in said urine sample, using said point-of-care (POC) device assembled in step b), wherein, preferably, said single-use test-strip is the single-use monoanalytical test-strip as defined further above and below.

In one embodiment, said steps b) and c) are performed in any order, i.e. step c) may be performed before step b), or said steps may be performed simultaneously.

In one embodiment, said test-strip holder unit is connected to said readout-meter device before or after said test-strip is inserted into said test-strip holder unit.

In one embodiment, a release mechanism is activated after said step d) for removing said test-strip from a receiving module of said readout-meter device or from said test-strip holder unit to avoid contact of the user with a urine sample.

In this aspect, said POC device, said measuring are as defined above.

In a further aspect, the present invention relates to a method of detecting and/or determining deficiency and/or overload of an analyte in a patient's body, said method comprising the steps:
- performing the method according to another embodiment of the present invention
- detecting and/or determining an analyte deficiency, if the detected concentration in said urine sample is lower than a physiologically adequate value for the patient, and detecting and/or determining an analyte overload, if the detected concentration is higher than a physiologically adequate value for the patient.

In one embodiment, said analyte is selected from cations of alkali metals, preferably selected from Na⁺, K⁺, and Li⁺, alkaline earth metals, preferably selected from Ca²⁺ and Mg²⁺, other metals, which are non-alkali metals and non-alkaline earth metals, preferably selected from Pb²⁺, Zn²⁺, and Cu²⁺, anions selected from halides, in particular Cl⁻ and I⁻, phosphates, and sulfates, and non-metal cations, preferably selected from NH₄⁺, H⁺, and creatinine⁺.

It should be noted that the quantitative determination of the concentration of an analyte occurs in an enzyme-free manner. Hence, no enzymes are used for such determination, and no products of an enzymatic reaction are measured/determined. Such determination is based on potentiometric measurements, i.e. it involves the measurement of differences in electric potential. It does not involve measurements of electric current.

The present inventors have provided for a simple, sensitive, non-invasive, quantitative and low-cost portable single-use monoanalytical test-strip for the quantitative determination of the concentration of an analyte in a patient's urine sample and for a non-invasive, quantitative, low-cost and portable point-of-care (POC) device for detecting and/or determining deficiency and/or overload of an analyte in a patient's body. The test-strip and the point-of-care device enable electrochemical measurements of urinary concentrations of an analyte, wherein said analyte is preferably selected from cations of alkali metals, preferably selected from Na⁺, K⁺, and Li⁺, alkaline earth metals, preferably selected from Ca²⁺ and Mg²⁺, other metals, which are non-alkali metals and non-alkaline earth metals, preferably selected from Pb²⁺, Zn²⁺ , and Cu²⁺, anions selected from halides, in particular Cl⁻ and I⁻, phosphates, and sulfates, and non-metal cations, preferably selected from NH₄⁺, H⁺, and creatinine⁺.

### DETAILED DESCRIPTION

The term "test-strip", as used herein, in a simple embodiment, is meant to refer to a substrate that is to be brought in contact with a sample, in order for the quantitative measurement of an analyte concentration to be performed, thus said test-strip being a sensing unit for a quantitative determination of an analyte. In one embodiment, said contacting of a test-strip with a sample is performed by dipping said test-strip into said sample. The test-strip may take any form that is suitable to be contacted with a patient's sample, such as a rectangular form, square form, circular form or oval form. In one embodiment, the test-strip is planar, having a planar electrically insulating substrate on which an electrode assembly has been applied. However, in other embodiments, the test-strip may also adopt other shapes and forms, such as a sheet or rod or tube, provided such form is suitable to accommodate a substrate on which an electrode array can be deposited. In one embodiment, said substrate is a planar substrate; in another embodiment, said substrate is a non-planar substrate. For example it may also be in the form of a sheet or rod or tube. In one embodiment, the electrodes of said electrode array are all arranged in a single plane on said substrate, or on said insulating layer, if present; in another embodiment, said electrodes do not necessarily have to be in a single plane, but may be arranged in different planes at an angle to each other. The only requirement is that the electrodes are arranged within the electrode array such that they can be brought in contact with urine when the test-strip is contacted with a urine sample.

In one embodiment, the test-strip according to the present invention is a single test-strip, on its own; in another embodiment, said test-strip forms part of an array of test-strips, such as may be arranged in a roll or on a disc, wherein, for each measurement, one test-strip may be used at a time. Hence, the present invention also envisages and relates to a plurality of test-strips in accordance with the present invention which are connected with each other. Hence the present invention also relates to an array of test-strips according to the present invention. In such array, each test-strip is intended for single use, but the entire array may be used for as many times as there are test-strips in such array. In one embodiment, such array of test-strips may be provided in the form of a cartridge or other dispensing device which allows the sequential use of test-strips. In one embodiment, within such array, the test-strips may be releasably connected with each other, such that, for example, if a test-strip is to be used for a measurement, it can be disconnected from the array and be used thereafter.

The term "single-use", as used herein, refers to the test-strip of the present invention being disposed after having been used once for measurement of an analyte concentration. Hence, the test-strip in accordance with embodiments of the present invention is a disposable test-strip.

The term "analyte-selective" as used herein, in the context of an electrode or a membrane, is meant to refer to an electrode or membrane that is selectively sensitive for a certain analyte. In one embodiment, such selective sensitivity of an electrode is achieved by applying an analyte-selective membrane on said electrode. In one embodiment, such analyte-selective membrane is produced by applying an analyte-selective membrane solution onto the surface of the respective electrode. The application may be done by dispensing, drop casting, screen printing, spin coating, inkjet printing or any other suitable deposition method. Such an analyte-selective membrane solution typically contains an analyte-specific carrier molecule such as an ionophor. The solution also typically contains a polymer and a solvent. Such solution may also and optionally contain other components, such as plasticizers, and/or a cation-exchanger salt. The analyte-selective solution may be prepared for example by dissolving all components in a suitable solvent. Suitable solvents are manifold, e.g. tetrahydrofurane, methanol or dimethylformamide. Once the analyte-selective membrane solution has been applied onto the surface of the electrode, the solvent is removed by drying, evaporation, etc., and what remains is a polymeric membrane layer which contains analyte-specific and -selective carriers.

In one embodiment, said analyte in "analyte-selective" is preferably sodium, and said sodium-selective carriers are typically crown ethers, calix(4)arenes, silacrown ethers and related macrocyclic hosts as well as noncyclic di- and tri-amides or derivatives from the monensin (carboxylic acid antibiotics) family. Examples of sodium-selective carriers are 4-tert-butylcalix(4)arenetetraacetic acid tetraethyl ester, 2,3:11,12-didecalino-16-crown-5; bis[(12-crown-4)methyl] dodecylmethylmalonate; bis[(12-crown-4)methyl] 2,2-didodecylmalonate; 4-octadecanoyloxymethyl-N,N,N',N'-tetracyclohexyl-1,2-phenylenedioxydiacetamide;
(N,N',N"-triheptyl-N,N',N"-trimethyl-4,4',4"-propylidynetris(3-oxabutyramide); N,N'-dibenzyl-N,N'-diphenyl-1,2-phenylenedioxydiacetamide); monensin methyl ester or monensin dodecyl ester.

In one embodiment, said sodium-selective carrier molecule is present in a percentage weight of from 0.5 % to 10%, preferably from 1% to 4%, in relation to the total weight of the analyte-selective membrane.

In another embodiment, said analyte in "analyte-selective" is preferably potassium, and said potassium-selective carriers are typically derivatives from the family of antibiotics, mono- and bis-15-crown-5 ethers. Examples of potassium-selective carriers are valinomycin, bis[(benzo-15-crown-5)-4'-ylmethyl] pimelate, and bis[(benzo-15-crown-5)-4'-ylmethyl] 2-dodecyl-2-methylmalonate, and 2-dodecyl-2-methyl-1,3-propanediyl bis[N-[5'-nitro(benzo-15-crown-5)-4'-yl]carbamate].

In one embodiment, said potassium-selective carrier molecule may be present in a percentage weight of from 0.5 % to 10%, preferably from 1% to 5%, in relation to the total weight of the analyte-selective membrane.

In another embodiment, said analyte in "analyte-selective" is preferably calcium, and said calcium-selective carriers are typically from the family of antibiotics, acyclic diamide derivatives, diazacrown ether rings, or phosphoric acid esters. Examples of calcium-selective carriers are 3,6-dioxaoctanediamide, N,N,N',N'-tetracyclohexyl-3-oxapentanediamide, bis[4-(1,1,3,3-tetramethylbutyl)phenyl)] phosphate, dodecyl phosphate, (1,7-di[2-(1-phenylazo) naphthyl]-1,4,7-trioxaheptane), and bis(octadecyl- 3-oxapentanediamide) 21-diazacrown ether. In one embodiment, said calcium-selective carrier molecule may be present in a percentage weight of from 0.5% to 10%, preferably from 1% to 5%, in relation to the total weight of the analyte-selective membrane.

In another embodiment, said analyte in "analyte-selective" is preferably magnesium, and said magnesium-selective carriers are typically di- and triamide, β-diketone derivatives, and double-substituted diazacrown ethers. Examples of magnesium-selective carriers are N,N'-diheptyl-N,N'-dimethyl-1,4-butanediamide, N,N"-octamethylene-bis(N'-heptyl-N'-methyl-methylmalonamide, N,N"-octamethylene-bis(N'-heptyl-N'-methylmalonamide), 4,13-[bis(N-adamantylcarbamoyl)acetyl]-1,7,10,16,tetraoxa-4,13-diazacyclooctadecane, and 1,2-bis(ditolylphosphine oxide)benzene).

In one embodiment, said magnesium-selective carrier molecule may be present in a percentage weight of from 0.5% to 10%, preferably from 1% to 5%, in relation to the total weight of the analyte-selective membrane.

In another embodiment, said analyte in "analyte-selective" is preferably chloride (Cl⁻), and said chloride-selective carriers are typically derivatives from the family of bis-thiourea compounds, organomercury compounds and alkyl-substituted metal porphyrins. Examples of chloride-selective carriers are dodecylbenzylmercury(II) chloride, 3,6-didodecyloxy-4,5-dimethyl-o-phenylene-bis(mercury chloride), 4,5-Dimethyl-3,6-dioctyloxy-o-phenylene-bis(mercurytrifluoroacetate), 2,7-di-tert-butyl-9,9-dimethyl-4,5-xanthenediamine, chloro(5,10,15,20-tetraphenylporphyrinato) manganese(III), chloro(5,10,15,20-tetraoctylporphyrinato)indium(III).

In one embodiment, said chloride-selective carrier molecule may be present in a percentage weight of from 0.5 % to 10%, preferably from 1% to 6%, in relation to the total weight of the analyte-selective membrane.

In another embodiment, said analyte in "analyte-selective" is preferably iodide (in form of I⁻, I₃⁻), and said iodide-selective carriers are typically derivatives from the family of vitamin B12, diquaternary ammonium salts, metallated phthalocyanines and porphyrines. Examples of iodide-selective carriers are N,N,N',N'-tetramethyl-N,N'-dioctadecylethylene-1,2-diammonium iodide, bis(salicylaldehyde)phenyl-diiminecobalt(III), titanium acetylacetonate, titanyl phthalocyanine, poly-tetrakis(p-aminophenyl)porphyrin, 9-mercuracarborand-3, and for I³⁻, 5,10,15,20-tetraphenylporphyrinato)manganese(III).

In one embodiment, said chloride-selective carrier molecule may be present in a percentage weight of from 0.5 % to 10%, preferably from 1% to 6%, in relation to the total weight of the analyte-selective membrane.

In another embodiment, said analyte in "analyte-selective" is preferably phosphate (in form of HPO₄²⁻, H₂PO₄⁻ or P0₄³⁻), and said phosphate-selective carriers are typically derivatives from the family of organotin compounds, mainly dibenzyltin dichlorides, Co(II) and Ni(II) complexes, mainly phthalocyanines, and cyclic polyamines without a metal center. Examples of phosphate-selective carriers are 3-decyl-1,5,8-triazacyclodecane-2,4-dione, bis(p-fluorobenzyl)tin dichloride, and bis(dibromophenylstannyl)methane. In one embodiment, said phosphate-selective carrier molecule may be present in a percentage weight of from 1% to 30%, preferably from 15% to 25%, in relation to the total weight of the analyte-selective membrane. In one embodiment, said phosphate-selective carrier molecule may be present in a percentage weight of from 1% to 10%, preferably from 2% to 5%, in relation to the total weight of the analyte-selective membrane.

In another embodiment, said analyte in "analyte-selective" is preferably sulfate, and said sulfate-selective carriers is typically 1,3-[Bis(3-phenylthioureidomethyl)]benzene. In one embodiment said sulfate-selective carrier molecule may be present in a percentage weight of from 0.5 % to 10%, preferably from 1% to 5%, in relation to the total weight of the analyte-selective membrane.

In another embodiment, said analyte in "analyte-selective" is preferably lithium (Li⁺), and said lithium ion-selective carriers are typically derivatives from the family of substituted 12-crown-4 to 15-crown-4, di- and triamides, and carboxylic polyethers. Examples of lithium ion-selective carriers are 6,6-dibenzyl-14-crown-4, decalino-14-crown-4, 7-tetradecyl-2,6,9,13-tetraoxatricyclo[12.4.4.01,14]docosane, 2,3-bis(diisobutylcarbamoylmethyl)-1,4,8,11-tetraoxacyclotetradecane, and cis-cyclohexane-1,2-dicarboxamides, N,N'-Diheptyl-N,N',5,5-tetramethyl-3,7-dioxanonanediamide.

In one embodiment, said lithium ion-selective carrier molecule may be present in a percentage weight of from 0.5% to 10%, preferably from 1% to 5%, in relation to the total weight of the analyte-selective membrane.

In another embodiment, said analyte in "analyte-selective" is preferably copper (Cu²⁺), and said copper ion-selective carriers are typically derivatives from the family of macrocyclic tetrathioethers, noncyclic dithiocarbamates and hydroxamates. Examples of copper ion-selective carriers are N-(m-nitrocinnamoyl),N-(p-chlorophenyl)hydroxylamine, N,N,N',N'-tetracyclohexyl-2,2'-thiodiacetamide, o-xylylene-bis(N,N-diisobutyldithiocarbamate), 2-(1,4,8,11-tetrathiacyclotetradec-6-yloxy)hexanoic acid, and tetraethylthiuram disulfide.
In one embodiment, said copper ion-selective carrier molecule may be present in a percentage weight of from 0.5% to 10%, preferably from 1% to 5%, in relation to the total weight of the analyte-selective membrane.

In another embodiment, said analyte in "analyte-selective" is preferably lead (Pb²⁺), and said lead ion-selective carriers are typically derivatives from the family of oxa- or dioxadicarboxylic acid diamides, amide substituted calix[4]arenes. Examples of lead ion-selective carriers are tert-butylcalix[4]arene-tetrakis(N,N-dimethylthioacetamide), S,S'-methylene-bis(N,N-diisobutyldithiocarbamate, diphenylmethyl-N-phenylhydroxamic acid, N,N-dioctadecyl-N',N'-dipropyl-3,6-dioxaoctane-1,8-diamide, nonylphenoxypoly(ethyleneoxy)ethanol, 3,6,9-triooxaundecane-1,11-diamide, N,N'-diheptyl-N,N',6,6-tetramethyl-4,8-dioxaundecanediamide, dicyclohexano-18-crown-6, tetrakis(2-pyridylmethyl)ethylenediamine, diphenylmethyl-N-phenylhydroxamic acid, and diazadibenzo-18-crown-6 ether.

In one embodiment, said lead ion-selective carrier molecule may be present in a percentage weight of from 0.5% to 10%, preferably from 1% to 5%, in relation to the total weight of the analyte-selective membrane.

In another embodiment, said analyte in "analyte-selective" is preferably zinc (Zn²⁺), and said zinc ion-selective carriers are typically derivatives from the family of alkylated thiuram disulfides, azaglutaric acid diamides. Examples of zinc-selective carriers are tetrabutylthiuram disulfide, N-benzyliminodiacetic acid bis(N-ethyl-N-cyclohexylamide) and potassium hydrotris(N-t-butyl-2-thioimidazolyl)borate.

In one embodiment, said zinc-selective carrier molecule may be present in a percentage weight of from 1% to 10%, preferably from 2% to 6%, in relation to the total weight of the analyte-selective membrane.

In another embodiment, said analyte in "analyte-selective" is preferably creatinine (Crea⁺), and said creatinine ion-selective carriers are typically derivatives from the family of crown ethers, α-, β-cyclodextrines, calixpyrroles, amino-pyridone, and amino-pyrimidones. Examples of creatinine ion-selective carriers are dibenzo-30-crown-10; tri-o-octyl-β-cyclodextrin; 2,6-di-o-dodecyl-β-cyclodextrin; 1-(5,7,7-trimethyl-2-(1,3,3-trimethylbutyl)-octyl)isocytosine. In one embodiment, the creatinine-selective carrier may be an ion-pair complex, such as creatinine tungstophosphate, creatinine molybdophosphate or creatinine picrolonate.

In one embodiment, said creatinine ion-selective carrier molecule may be present in a percentage weight of from 0.5% to 10%, preferably from 1% to 5%, in relation to the total weight of the analyte-selective membrane.

In one embodiment, prior to the potentiometric measurement, the creatinine has to be protonated to form a creatinium ion by adjusting the pH of said sample by addition of a suitable buffer. The term "creatinine-selective" is meant to encompass a selectivity for creatinine, irrespective and independent of the protonation state of said creatinine. Hence, a creatinine-selective electrode is selective for either creatinine in non-protonated form, or in protonated form, or both. In one embodiment, a suitable pH is below 5, and suitable buffers are manifold. e.g. they may be acetate, citrate, or phosphate.

In another embodiment, said analyte in "analyte-selective" is preferably ammonium, and said ammonium-selective carriers are typically from the family of antibiotics, substituted glycol dibenzyl ethers or cyclic 19-crown-6 ethers. Examples of ammonium-selective carriers are nonactin, monactin, a mixture thereof, 2,5-dibenzyloxy-2,5-dimethylhexane, and 12,12'-methylenedi-11,13-dioxatricyclo[4.4.3.01,6]tridecane.

In one embodiment, said ammonium-selective carrier molecule may be present in a percentage weight of from 0.5 % to 10%, preferably from 1% to 5%, in relation to the total weight of the analyte-selective membrane. In another embodiment, if said ammonium-selective carrier molecule acts also as plasticizer, as it is the case for glycol dibenzyl ethers, said ammonium-selective carrier molecule may be present in a percentage weight of from 30 % to 80%, preferably from 50% to 75%, in relation to the total weight of the analyte-selective membrane.

In another embodiment, said analyte in "analyte-selective" is preferably hydrogen ion (in form of H⁺, H₃O⁺), and said hydrogen ion-selective carriers are typically derivatives from the family of cyclic and acyclic amines, and perflorocabonylamines. Examples of hydrogen ion-selective carriers are tridodecylamine, 4-nonadecylpyridine, octadecyl isonicotinate, 9-(diethylamino)-5-octadecanoylimino-5H-benzo[a]phenoxazine, 4'-(dipropylamino)azobenzene-2-carboxylic acid octadecylester.

In one embodiment, said hydrogen ion-selective carrier molecule may be present in a percentage weight of from 1% to 20%, preferably from 1% to 10%, in relation to the total weight of the analyte-selective membrane.

Polymers or mixture of polymers, that may be used for the preparation of the membrane solution (from which a polymer matrix is generated) and which subsequently function as a polymer matrix in the membrane are manifold and, in one embodiment, are selected from polyvinyl chloride, polystyrene, polyacrylates, polycarbonates, polyesters, polyamides, polyurethanes, polyvinylidene chloride, polyvinyl acetate, polyvinyl alcohols, polysiloxanes, polymetacrylates, silicone elastomers, cellulose esters. Possible are also fluorous phases of these polymers to realize extremely hydrophobic analyte-selective electrode (ASE) membranes, e.g. ion-selective electrode (ISE) membranes. In one embodiment, the polymers are preferably of high molecular average weight to guarantee an inert character of the membrane. In one embodiment, the percentage weight of polymeric material is from 20 % to 40 % in relation to the total weight of the analyte-selective membrane.

In one embodiment, one or several plasticizers are included in the membrane solution. Their role is to make the membrane softer and resistant to mechanical stress. In one embodiment, plasticizer(s) that may be used in the membrane solution, may be selected from o-nitrophenyl-octylether, bis(2-ethylhexyl)adipate, bis(2-ethylhexyl)sebacate, dioctyl sebacate, dioctylphenyl phosphonate, dimethyl phthalate, dibutyl phthalate, dioctyl phthalate, hexamethylphosphoramide, bis(1-butylpentyl)adipate, chloroparaffin. In one embodiment, the plasticizer is present in a percentage weight sufficient to solvate analyte-selective carrier in the polymeric material. In one embodiment, the weight ratio plasticizer to analyte-selective carrier is 10:1 to 100:1. In one embodiment, the weight ratio of plasticizer:polymer in typical plasticizer:polymer mixtures is in a range of from 1:1 to 4:1. In one embodiment, the percentage weight of plasticizer is from 40 % to 80 % in relation to the total weight of the membrane.

Optionally, and in certain embodiments, in particular when the analyte-selective carrier is a neutral molecule, a cation-exchanger salt may be added in the polymer matrix. In one embodiment, such salt is composed from a large negatively charged organic molecule and a small cation. Its function is to help maintaining permselectivity of the membrane by complementing each cationic analyte captured by the membrane by the large negatively charged organic molecule, e.g. a lipophilic anion, and exchanging in the membrane only ions with the same sign.
Examples for such cation exchanger salts are potassium or sodium tetrakis[3,5-bis(trifluoromethyl)phenyl]borate, potassium or sodium tetrakis(p-chlorophenyl)borate, potassium or sodium tetrakis[3,5-bis(1,1,1,3,3,3-hexafluoro-2-methoxy-2-propyl)phenyl]borate.

In one embodiment, typical molar ratios of cation-exchanger salt:analyte selective carrier mixtures are in a range of from 1:10 to 1:2. In one embodiment, the percentage weight of cation-exchanger salt is from 0.1% to 2% in relation to the total weight of the membrane.
It is however, clear that with respect to the polymer matrix and its different components, these different components of the polymer matrix will be present and used in such amounts that their total will be 100 % by weight.

Optionally, and in some embodiments, before applying the analyte-selective solution onto the surface of the respective electrode, or a polymeric material comprising Ag/AgCl/Cl⁻ or a lipophilic salt, on the reference electrode, an "inner contact layer" material (also called ion-to-electron transducer) may be coated on said electrode. Without wishing to be bound by any theory, such inner contact layer has the function of avoiding the formation of capacitive layers at the electrode/membrane interface. This may enhance the sensitivity and accuracy of the analyte detection. Examples of materials suitable as "inner contact layer" are conducting polymers such as polyaniline, poly(3,4-ethylenedioxythiophene), poly(3-octylthiophene), polypyrrole, polyaniline, conductive carbon based materials such as graphene, carbon nanotubes, graphene, graphene oxide, reduced graphene oxide or metal nanoparticles. In one embodiment, they may be solubilized or dispersed in a suitable solvent and applied onto the surface of the said electrode by dispensing, drop-casting, screen printing, spin coating, inkjet printing or any other suitable deposition method. Once the solution/dispersion has been applied onto the surface of the electrode, the solvent is removed by drying, evaporation, etc., and what remains is an "inner contact layer" on which afterwards the analyte-selective membrane is applied.

In one embodiment, the "inner contact layer" material may, optionally, also be directly included in the polymeric membrane solution of the analyte-selective membrane or polymeric material applied on the reference electrode.

In some embodiments, although the electrodes should be in electrical contact with the liquid sample, e.g. urine sample, it may be useful to prevent the electrode array and electrical leads from coming into contact with larger molecules or urinary components, such as proteins, which may interfere with and have a negative impact on the quality of the determination(s) of concentration(s). Such worsening process, due to larger molecules is called "biofouling". Therefore, in some embodiments, optionally, a covering membrane may applied on the portion of said test-strip which is intended to come into contact with said urine sample. An example of such a suitable covering membrane is a polycarbonate material, e.g. those sold under the Trade Mark "Nucleopore" allowing the trapping of large interfering molecules.

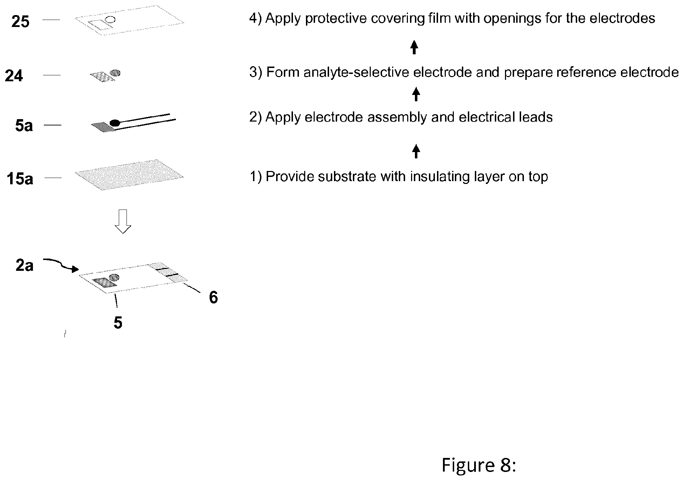

Furthermore, in some embodiments, at the end of the fabrication of a test-strip, a suitable dielectric protective layer, e.g. a plastic insulating material having openings for the electrodes can be applied on the test-strip (Figure 8) to build up a test-strip (2a) having an exposed electrode array (5) and terminal interface (6), for omitting contamination of electrical leads during storage and processing and for the commodity of use for the user.

It should be noted that, in one embodiment, the test-strip in accordance with the present invention is not a rod-like structure, but is or comprises a planar electrically insulating substrate. In one embodiment, the quantitative determination of an analyte concentration is not based on colorimetric or amperometric measurements. In one embodiment, it is based on potentiometric measurements. Moreover, in one embodiment, the quantitative determination of an analyte concentration according to the present invention does not involve the use of enzymes, nor any oxidation/reduction reaction nor any hydrolysis of an analyte.

In embodiments according to the present invention, the single-use monoanalytical test-strip is to be used in connection with a point-of-care device in accordance with the present invention for detecting deficiency and/or overload of an analyte. In one embodiment, a single-use monoanalytical test-strip is connected to the readout-meter device by means of the receiving module of the readout-meter device. In one embodiment, a single-use monoanalytical test-strip is connected to an interface of a test-strip holder unit, wherein said test-strip holder unit is connected to said receiving module of the readout-meter device. For that purpose, the single-use test-strip has a suitable interface for electrically connecting the electrode assembly of said test-strip to said readout-meter device which forms part of the point-of-care device of the present invention, or to said test-strip holder unit that is optionally interposed between said test-strip and said readout-meter device. Such interface may take any suitable form and may, in one embodiment, be a set of electrical contacts coming from the electrode assembly on the planar substrate. Such interface may, for example, take the form of a plug, with the electrical contacts forming part of said plug. The interface is suitable for electrically connecting the electrode assembly to a readout-meter device or to a test-strip holder unit of a non-invasive point-of-care device for determining deficiency and/or overload of an analyte, wherein such point-of-care device comprises a readout-meter device and optionally a test-strip holder unit for receiving an interface of a single-use test-strip such as a single-use monoanalytical test-strip according to the present invention, e.g. in the form of a recess or well or slit, for receiving the interface of the test-strip. In one embodiment, such test-strip holder unit may take the form of a socket. Typically, the test-strip holder unit is suitable to accommodate the interface of the single-use test-strip.

The term "monoanalytical test-strip", as used herein, relates to a test-strip which can be used to analyze a sample with regard to only one analyte of interest. A monoanalytical test-strip according to the present invention has an analyte-selective working electrode for one analyte only, and does not have analyte-selective working electrodes for other analytes. The monoanalytical test-strip may comprise a second analyte-selective working electrode, which is a control working electrode, selective for the same analyte as the first analyte-selective working electrode. A monoanalytical test-strip according to the present invention is preferably dipped into a urine sample to analyze said urine sample with regard to an analyte of interest. In one embodiment, said monoanalytical test-strip is a monoanalytical dipstick test-strip which is used by dipping said test-strip into a urine sample. A monoanalytical test-strip according to the present invention does not have electrodes for different analytes, but may be combined or used in conjunction with other test-strips that have electrodes for other analytes to compose an array of test-strips with different analyte-selectivities.

The term "second analyte-selective working electrode", as used herein, relates to an optional control working electrode of a monoanalytical test-strip according to the present invention. Said second analyte-selective working electrode is selective for the same analyte as the first analyte-selective working electrode of said monoanalytical test-strip.

The term "neutral electrode", as used herein, relates to an optional control electrode of a monoanalytical test-strip according to the present invention. A neutral electrode according to the present invention is for measuring and determining interferences.

The abbreviation "EMF", as used herein, refers to the electromotive force, which essentially refers to a difference in potential between two electrodes. Such electromotive force is quantitatively related via the Nernst equation to the corresponding analyte concentration (via activity) in a sample. Typically, the potentiometric signal or measurement that can be taken from a test-strip according to the present invention is an electromotive force which can then be related/converted into an analyte concentration. The abbreviation "WE", as used herein, refers to a working electrode, the abbreviation "RE" refers to a reference electrode, and the abbreviation "NE" to a neutral electrode.

The substrate that is comprised by a test-strip of the present invention is preferably made of an electrically insulating material. In one embodiment, such electrically insulating material is a dielectric material, such as plastic, ceramic, alumina, paper, cardboard, rubber, textile, carbon-based polymers, such as polypropylene, fluoropolymers, such as Teflon, silicon-based substrates, such as glass, quartz, silicon nitride, silicon oxide, silicon based polymers such as polydimethoxysiloxane, semiconducting materials such as elemental silicon. The substrate may optionally be coated with an electrically insulating layer. Such electrically insulating substrate is preferably made of a dielectric material, preferably selected from organic dielectric materials such as polyimide, polycarbonate, polyvinyl chloride, polystyrene, polyethylene, polypropylene, polyester, polyethylene terephthalate, polyurethane, polyvinylidene fluoride or inorganic dielectric materials such as silicium dioxide. In one embodiment, the electrode assembly that is applied on the substrate or on said electrically insulating substrate, if present, is part of and/or forms part of the surface of the substrate and is suitable to be brought in contact with a patient's sample, such as a urine sample. If an electrically insulating layer is present on the substrate, the electrode assembly is preferably located and applied on such electrically insulating layer.

The test-strip, in accordance with embodiments of the present invention is a single-use test-strip, which means that after having been used once for measurements of an analyte concentration, it is disposed. Hence, the test-strip in accordance with embodiments of the present invention is a disposable test-strip. It is to be used in conjunction with a non-invasive point-of-care (POC) device in accordance with the present invention for determining deficiency and/or overload of an analyte in a patient's body. In one embodiment, said disposable test-strip is a single-use monoanalytical test-strip. In one embodiment, said disposable test-strip is a single-use monoanalytical test-strip which is preferably used by dipping said single-use monoanalytical test-strip into a urine sample. In one embodiment, said disposable test-strip is a single-use monoanalytical dipstick test-strip which is used by dipping said single-use monoanalytical dipstick test-strip into a urine sample.

The term "non-invasive point-of-care (POC) device", as used herein, relates to a device for quantitative potentiometric determination of the concentration of an analyte in a urine sample. A POC device of the present invention comprises a readout-meter device as an analyzer unit for the quantitative and selective potentiometric measurement of an analyte concentration in a urine sample. A POC device optionally comprises a test-strip holder unit which connects a test-strip to said readout-meter device of the POC device. The POC device is combined with a single-use test-strip as sensing unit for the quantitative determination of an analyte in urine, optionally by means of said test-strip holder unit which is interposed between said readout-meter device of said POC device and said test-strip. A POC device of the present invention further comprises a measurement mechanism and/or a release mechanism. In one embodiment, said measurement mechanism and/or said release mechanism are part of the readout-meter device. In another embodiment, said measurement mechanism and/or release mechanism are part of said test-strip holder unit.

The term "readout-meter device", as used herein, relates to a device suitable and configured to quantitatively measure electrical signals received from a sample-exposed single-use test-strip connected to the readout-meter device via a receiving module of said readout-meter device, wherein optionally a test-strip holder unit is interposed between said receiving module of said readout-meter device and said test-strip. The readout-meter device according to the present invention is an electrometric device which is either a direct reading circuit or a null-balance potentiometric circuit. In many of the embodiments of the present invention, said readout-meter device is a multi-use digital potentiometric readout-meter for quantitatively determining an analyte in a patient's urine sample. The readout-meter device is part of the POC device and is configured to calculate the concentration of an analyte in the sample based on the electrical signals and calibration information, and is also configured to output, e.g. display the results to a user. In one embodiment, such readout-meter device comprises a measuring unit such as a software, i.e. a controller that is configured to measure electrical signal(s) received from said test-strip or to measure potential differences in the electrode assembly of said test-strip, and to calculate the concentration of an analyte based on such electrical signal(s). In one embodiment, said calibration information is initially determined for a set of test-strips and saved in a measuring unit such as a software to perform calculations of analyte concentrations later on. In one embodiment, said measuring unit is preferably triggered by a measurement mechanism. In some embodiments, for exerting control over a measurement, the measurement mechanism may be configured for this purpose or the measuring unit or both. Furthermore, the readout-meter device comprises an output unit connected to the controller for outputting results for inspection by a user. In one embodiment, such readout-meter device further comprises a communication unit, for example a USB and/or wireless port for exchanging data with an external computer or network. The readout-meter device has an interface part for receiving the test-strip or the test-strip holder unit. In one embodiment, the readout-meter device comprises a measurement mechanism and a release mechanism.

The term "receiving module", as used herein, relates to a receiving module of a readout-meter device for receiving an interface of a test-strip or a test-strip holder unit and for establishing electrical contact between said readout-meter-device and an electrode assembly of said test-strip, thus allowing the detection and transmission of electrical signal(s) from said test-strip to said readout-meter-device, optionally via a test-strip holder unit, wherein said receiving module has electrical connectors for separately contacting each electrode via said interface of said test-strip or said test-strip holder unit. In one embodiment, said receiving module is in the form of a slit, recess or well or other suitable form allowing to establish a connection to the interface of said test-strip or said test-strip holder unit. In one embodiment, said receiving module may be configured as an edge-connector pair or a pin-and-socket-pair. In one embodiment, a single-use monoanalytical test-strip according to the present invention is inserted into said receiving module of said readout-meter device by way of said interface of said single-use monoanalytical test-strip, thus establishing electrical contact between said electrode assembly of said test-strip and said readout-meter device. In one embodiment, a single-use monoanalytical test-strip according to the present invention is inserted into said test-strip holder unit which is connected to said receiving module of said readout-meter device by way of said interface of said single-use monoanalytical test-strip, thus establishing electrical contact between said electrode assembly of said test-strip and said readout-meter device via said test-strip holder unit. In one embodiment, said readout-meter device has a receiving module which is capable of receiving an interface of a single-use test-strip. In one embodiment, said readout-meter device has a receiving module which is capable of receiving an interface of a test-strip holder unit. In one embodiment, said readout-meter device has a receiving module which is capable of receiving an interface of a single-use test-strip, and has a receiving module which is capable of receiving an interface of a test-strip holder unit. In one embodiment, said readout-meter device has a receiving module which is capable of receiving an interface of a single-use test strip and/or a test-strip holder unit.

The term "interface", as used herein, relates to a first interface which is part of a receiving module of a readout-meter device, wherein said first interface is capable of establishing a contact to a second interface which is part of an optional test-strip holder unit capable of connecting to said first interface of said receiving module of said readout-meter device, and wherein said first interface is capable of establishing a contact to an interface of a test-strip referred to as a fourth interface. The term also relates to a third interface which is part of the test-strip holder unit capable of connecting to a fourth interface which is part of a test-strip. Said fourth interface which is part of said test-strip may be connected to said third interface of said test-strip holder unit or may also be directly connected to said first interface which is part of said receiving module of said readout-meter device.

The term "communication unit", as used herein, relates to an optional unit of the point-of-care (POC) device for transmitting results wired and/or wireless to a reading unit or network, for example a software app developed for data management and for easy communication between user and doctor, or a server connected to a hospital information system (HIS) or laboratory information system (LIS). In one embodiment, such communication unit comprises an USB port and/or a bluetooth module.

The term "measurement mechanism", as used herein, relates to a feature of the POC device that is used to initiate and, preferably, conduct the measurement of an analyte concentration. An exemplary measurement mechanism is a measure button, such as a push button, a press button, a touch button, or a digital control button, a lever, a second pressure point of a different button of the POC device or pressing a button of the POC device for a second time, or a voice-controlled means to initiate the measurement. The term "measurement mechanism", as used herein, may also relate to an automatic initiation of the measurement at a defined point of time, after the sensing unit senses a sample. In one embodiment, said measurement mechanism preferably operates in conjunction with a measuring unit to conduct a measurement.

The term "time-controlled manner", as used herein, relates to a mechanism of performing a measurement, wherein a measurement, after having been started, preferably at a defined point in time, is subsequently performed over a defined duration, wherein said defined duration is a measurement period. In one embodiment, the mean value is calculated for values acquired during a measurement period. In one embodiment, said mean value is displayed by means of the POC device. In one embodiment, said mean value is used for subsequent calculations of analyte concentration and/or body status of said analyte.

The term "measure button", as used herein, relates to an exemplary measurement mechanism, which is a button at the POC device that initiates a measurement when pushed (push button), pressed (press button), or touched (touch button). The term also comprises a digital control button such as a displayed button icon on a touchscreen.

The term "to initiate a measurement", when used in conjunction with a measurement mechanism, refers to an action whereby a measurement is started at a defined point in time, namely when the measurement mechanism is actuated.

The term "to conduct a measurement", when used in conjunction with a measurement mechanism, refers to an action whereby a measurement, after having been started at a defined point in time, is subsequently performed until its completion, and such performing includes control over various measurement parameters, such as the duration of measurement period or values' variance of the received electrical signals in a defined measurement period. In one embodiment, a measurement is conducted in a time-controlled manner. In one embodiment, a measurement is preferably conducted by a measurement mechanism in conjunction with a measuring unit, as defined further above. In some embodiments, for exerting control over a measurement, the measurement mechanism may be configured for this purpose or the measuring unit or both.

The term "release mechanism", as used herein, relates to a means or a mechanism that allows to release a test-strip from a receiving module of a readout-meter device or from a test-strip holder unit. An exemplary release mechanism is a button, such as a push button, a press button, a touch button, or a digital control button, a lever, a second pressure point of a button of the POC device or pressing a button of the POC device for a second time, e.g. a measure button, or a voice-controlled release mechanism. A release mechanism may be part of a readout-meter device or of a test-strip holder unit. The term "release mechanism", as used herein, may also relate to an automatic release of the test-strip, when the measurement is finished.

The term "second pressure point of a button", as used herein, relates to a feature of a button which has a certain function to evoke also a second, different function, wherein said second, different function can be initiated by pressing said button at a second pressure point of said button. For example, a button may be pushed to a defined first level to evoke a first function of said button, and may be pushed to a defined second level to evoke a second function of said button, wherein said first and said second level are different. For example, a second pressure point of a button may be activated by pressing, pushing, or touching said button at said second pressure point. The term may also relate to a second position of a lever which allows to activate a second function of said lever. The term also comprises a second function of a button, wherein said button can be activated to perform a different function depending on the length of time that said button is activated, for example, by pushing, pressing or touching.

The term "pressing a button for a second time", as used herein, relates to a feature of a button which has a certain first function to evoke also a second, different function, wherein said second, different function can be initiated by pressing said button for a second time. The term also comprises a feature of a button, wherein a button can be triggered to evoke different functions depending on the length of time said button is triggered by means of pressing, pushing, touching, or the like.

The term "voice-controlled", as used herein, relates to a mechanism that allows to control the POC device by voice. Voice-control is an optional feature of the POC device enabled by an optional voice-control unit. In one embodiment, said optional voice-control unit can be used to start a measurement, i.e. as measurement mechanism, and/or to release a test-strip from the test-strip holder unit, i.e. as release mechanism.

The term "test-strip holder unit", as used herein, refers to a unit which is optionally a part of a POC device according to the present invention, and electrically connects a digital readout-meter device with a single-use test-strip. The test-strip holder unit according to the present invention comprises electrical leads which allow to separately connect each of the electrical leads of a test-strip or a test-strip array with a digital readout-meter device. The test-strip holder unit further comprises an interface part for receiving a test-strip or a test-strip array and an interface part to connect to the receiving module of the readout-meter. By using a release mechanism, a test-strip or a test-strip array can be released from the test-strip holder unit. The test-strip holder unit is detachable, for example by plugging/unplugging, from the readout-meter device, if necessary, e.g. for cleaning. The test-strip holder unit may have any size and shape such as a rectangular shape, a rod-like shape, or a tube-like shape. The test-strip holder unit has a main body which may be rigid or flexible, wherein preferably, for the commodity of use for the user, said main body is a rigid main body. Said test-strip holder unit comprises at least one interface suitable for connecting to an interface of a single-use test-strip or a single-use test-strip array, and comprises one interface suitable for connecting to a receiving module of a readout-meter device. In one embodiment, said test-strip holder unit may take the form of a socket or have a rotable port for connecting to a readout-meter device.

The term "potentiometric", as used herein, relates to the measurement of differences in electric potential of electrodes, e.g. the potential difference between a reference electrode and an analyte-selective working electrode, under the conditions of zero (or negligible) current. A potentiometric measurement does not involve measurement of electric current, and does not involve use of enzymes and measuring products of enzymatic reactions, nor does the quantitative determination involve any oxidation/reduction reaction or any hydrolysis of an analyte. A measurement achieved with a POC device of the present invention and a test-strip according to the present invention is a potentiometric measurement. A potentiometric measurement according to the present invention is not based on a colorimetric or amperometric measurement. Typically, the potentiometric signal or measurement that can be taken from a test-strip according to the present invention is an electromotive force which can then be related/converted into an analyte concentration.

The term "potentiometric measurement", as used herein, in one embodiment, relates to the measurement of an analyte concentration by means of differences in potential of an analyte-selective working electrode and a reference electrode of a test-strip, wherein a reference electrode has a constant potential and an analyte-selective working electrode has a potential that develops following Nernst-linearity in relation to the amount of said analyte in a sample. In one embodiment, such a measurement is performed by contacting a single-use potentiometric test-strip, which is or was in contact with a urine sample, directly or via a test-strip holder unit to a digital readout-meter device of the present invention's POC device, thereby measuring the potential difference between the two electrodes, amplifying the signal, and converting the generated analog signal to a digital signal.

The term "deficiency", as used herein, relates to a disorder of a human body or an animal body, which is characterized by a pathological decrease, i.e. deficiency, of a certain analyte in the body. The deficiency may derive from low intake of such analyte or increased loss of such analyte.

The term "overload", as used herein, relates to a disorder of a human body or an animal body, which is characterized by a pathological increase, i.e. overload, of a certain analyte in the body. The overload may derive from high intake of such analyte or decreased loss of such analyte.

The term "physiologically adequate value" or "physiologically adequate value for the patient", as used herein, relates to a value of an analyte concentration which is within the range of values that may be measured in humans or animals that do not have a deficiency or overload of said analyte. In one embodiment, such a physiologically adequate value may depend on the constitution of the human or animal. In one embodiment, such a physiologically adequate value may depend on the age, sex, health condition, or nutrition of a human or animal. In one embodiment, said physiologically adequate value of Na²⁺ may be in the range of 40 mM to 220 mM, said physiologically adequate value of K⁺ may be in the range of 25 mM to 125 mM, said physiologically adequate value of Ca²⁺ may be in the range of 1 mM to 10 mM, said physiologically adequate value of Cl⁻ may be in the range of 110 mM to 250 mM, said physiologically adequate value of phosphate may be in the range of 1 mM to 50 mM, and said physiologically adequate value of ammonium may be in the range of 3 mM to 65 mM.

The term "analyte", as used herein, refers to a substance or molecule of the body of a human patient or an animal, which is to be monitored, measured, or determined by concentration in the urine of said human patient or animal. An analyte, e.g. a metal, may be present in an ionic form or in an elemental form. Said analyte is selected from any substance or molecule that is detectable in the urine of a human or an animal, preferably selected from cations of alkali metals, alkaline earth metals, of other metals, which are non-alkali metals and non-alkaline earth metals, preferably selected from lead, copper, and zinc, anions selected from halides, in particular chloride and iodide, phosphates, and sulfates, and non-metal cations selected from ammonium, hydrogen, and creatinine. In one embodiment, said analyte is preferably selected from ions of sodium (Na⁺), potassium (K⁺), calcium (Ca²⁺), magnesium (Mg²⁺), chloride (Cl⁻), and phosphate (in form of HPO₄²⁻, H₂PO₄⁻ and PO₄³⁻), lithium (Li⁺), lead (Pb²⁺), copper (Cu²⁺), zinc (Zn²⁺), creatinine (Crea⁺), ammonium (NH₄⁺), hydrogen ion (H⁺), iodide (I⁻), and sulfate (in form of HSO₄⁻ and SO₄⁻). In one embodiment, said analyte is preferably selected from ions of sodium (Na⁺), potassium (K⁺), calcium (Ca²⁺), magnesium (Mg²⁺), chloride (Cl⁻), and phosphate (in form of HPO₄²⁻, H₂PO₄⁻ and/or PO₄³⁻). In one embodiment, said analyte is preferably selected from ions of lithium (Li⁺), lead (Pb²⁺), copper (Cu²⁺), zinc (Zn²⁺), creatinine (Crea⁺), ammonium (NH₄⁺), hydrogen ion (H⁺), iodide (I⁻), and sulfate (in form of HSO₄⁻ and/or SO₄²⁻).

The term "alkali metals", as used herein, relates to chemical elements of the first group of elements in the periodic system, including chemical elements lithium (Li), sodium (Na), potassium (K), rubidium (Rb), caesium (Cs), and francium (Fr), and ions thereof.

The term "alkaline earth metals", as used herein, relates to chemical elements of the second group of elements in the periodic system, including chemical elements beryllium (Be), magnesium (Mg), calcium (Ca), strontium (Sr), barium (Ba), and radium (Ra), and ions thereof.

The term "patient", as used herein, refers to a human or an animal.

The single-use test-strip and the point-of-care (POC) device in accordance with the present invention are low-cost devices and extremely easy to handle and can therefore also be used by non-medical staff and patients. The devices are pocket-type portable, and they can be used in a non-invasive manner and are therefore providing a good basis for optimal patient compliance. This is, for example, particularly advantageous in children because the need for blood-taking is obviated. Furthermore, the devices and the methodology according to the present invention provide immediate analysis result, reduce the time as well as the technical and logistic complexity and the cost of routine clinical analysis and thereby greatly facilitate the analysis of an analyte in a patient's urine sample.

### BRIEF DESCRIPTION OF THE FIGURES

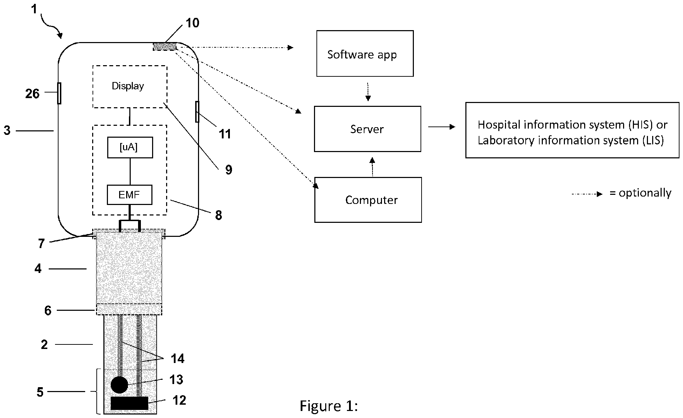

The present invention is now further described by reference to the following figures, wherein
**Figure 1** shows a schematic representation of an embodiment of a point-of-care (POC) device for detecting deficiency and/or overload of an analyte in a patient's body; EMF = electromotive force; uA = concentration of an analyte in a urine sample.
   1 = POC device,
   2 = single-use test-strip, e.g. single-use monoanalytical test-strip,
   3 = digital readout-meter device,
   4 = test-strip holder unit,
   5 = electrode assembly,
   6 = interface for electrical connection of test-strip with test-strip holder unit,
   7 = interface for electrical connection of test-strip holder unit with readout-meter device,
   8 = amplifier and controller,
   9 = display,
   10 = communication unit,
   11 = measure button to start measurement,
   12 = reference electrode,
   13 = analyte-selective working electrode,
   14 = electrical leads,
   26 = release button to release test-strip from test-strip holder unit.

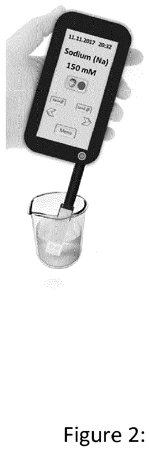

**Figure 2** shows an exemplary embodiment of a point-of-care (POC) device for quantitative determination of an analyte concentration in a urine sample consisting of a single-use test-strip, for example a single-use monoanalytical test-strip or a single-use monoanalytical dipstick test-strip, a test-strip holder unit, and readout-meter device displaying exemplary analysis results.

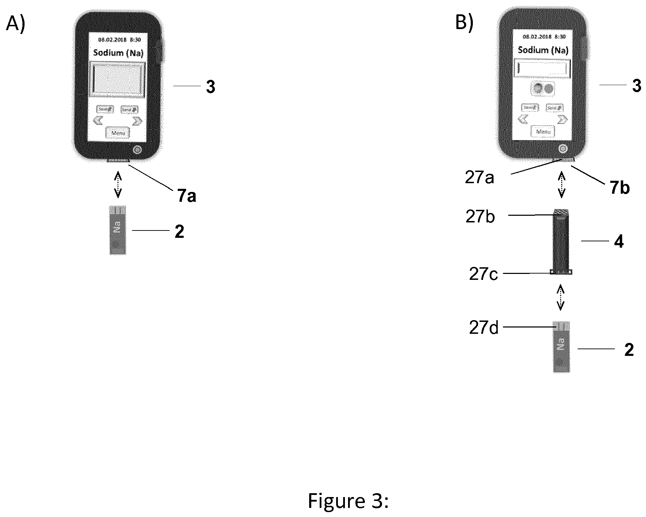

**Figure 3** shows an exemplary embodiment of a point-of-care (POC) device for quantitative determination of an analyte concentration in a urine sample comprising
   A) a single-use test-strip and a readout-meter device having a receiving module with an interface for receiving an interface of a single-use test-strip,
   B) a single-use test-strip, a test-strip holder unit, and a readout-meter device with a first interface for receiving a second interface of said test-strip holder unit contacted via a third interface with a fourth interface of a single-use test strip, wherein
      2 = single-use test-strip
      3 = readout-meter device
      4 = test-strip holder unit
      7a = receiving module for receiving interface of a single-use test-strip
      7b = receiving module for receiving interface of a test-strip holder unit
      27a = first interface as part of the receiving module of the readout-meter device for contacting second interface (27b) or fourth interface (27d)
      27b = second interface as part of the test-strip holder unit for contacting the interface of the receiving module (27a)
      27c = third interface as part of the test-strip holder unit for contacting the test-strip (27d)
      27d = fourth interface as part of the single-use test strip for contacting the interface of the receiving module directly (27a), or indirectly via the test-strip holder unit by contacting the third interface (27c).

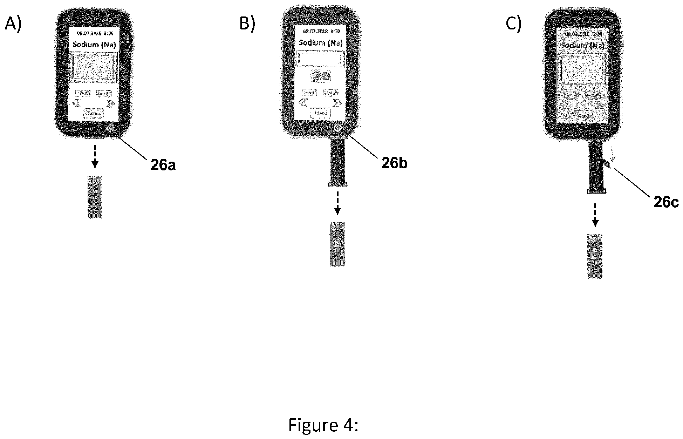

**Figure 4** shows an exemplary embodiment of a point-of-care (POC) device for quantitative determination of an analyte concentration in a urine sample comprising a release mechanism and
   A) a single-use test-strip and readout-meter having a release button (26a),
   B) a single-use test-strip, a test-strip holder unit, and a readout-meter device having a release button (26b),
   C) a single-use test-strip, a test-strip holder unit equipped with a release button in form of a lever (26c), and a readout-meter device,
      26a = release button to release a single-use test-strip from receiving module of the readout-meter
      26b = release button to release a single-use test-strip from the test-strip holder
      26c = release button in form of a lever placed on the test-strip holder unit to release a test-strip from the test-strip holder unit.

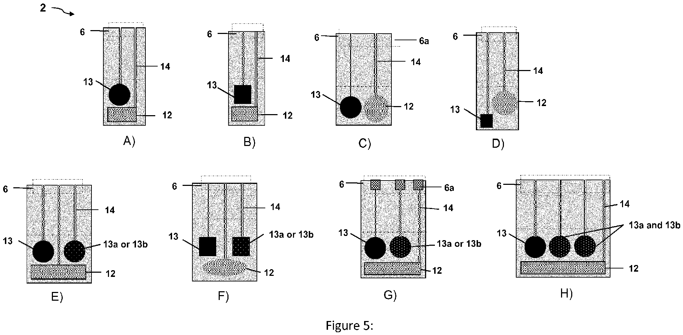

**Figure 5** shows top views of exemplary embodiments of test-strips with exemplary possible patterns of an electrode array and electrical leads applied on an insulating layer
   A) exemplary round shape of working electrode + rectangular shape of reference electrode;
      2 = single-use test-strip,
      6 = interface for electrical connection,
      12 = reference electrode,
      13 = analyte-selective working electrode,
      14 = electrical leads;
   B) exemplary square shape of analyte-selective working electrode + rectangular shape of reference electrode;
   C) exemplary round shape of analyte-selective working electrode + round shape of reference electrode;
   D) exemplary square shape of analyte-selective working electrode + round shape of reference electrode
   E) exemplary round shape of analyte-selective working electrode + rectangular shape of reference electrode + second round shape control working electrode or neutral electrode;
      13a or 13b = second analyte-selective electrode for control measurement or neutral electrode for determining interferences;
   F) exemplary square shape of working electrode + oval shape of reference electrode + second square shape working electrode or neutral electrode;
   G) exemplary round shape of analyte-selective working electrode + rectangular reference electrode + second round shape control working electrode or neutral electrode;
      6a = exemplary contact paths at the end of electrical leads for contacting readout-meter;
   H) exemplary round shape of working electrode + rectangular shape of reference electrode + second round shape of control working electrode + round shape neutral electrode.

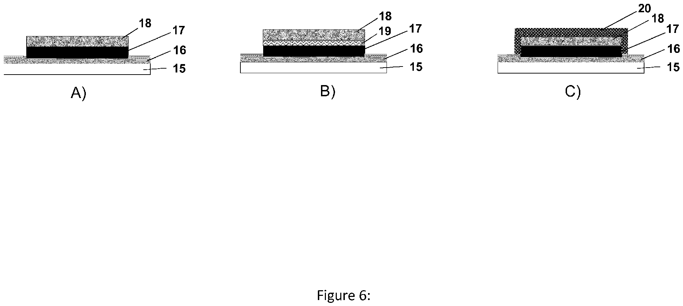

**Figure 6** shows cross-sectional views of an exemplary analyte-selective electrode
   A) without "inner contact layer"
      15 = substrate,
      16 = insulating layer,
      17 = conductive layer,
      18 = analyte-selective membrane,
   B) with "inner contact layer"
      15 = substrate,
      16 = insulating layer,
      17 = conductive layer,
      18 = analyte-selective membrane,
      19 = optionally, "inner contact layer" (transducer),
   C) with "covering membrane"
      15 = substrate,
      16 = insulating layer,
      17 = conductive layer,
      18 = analyte-selective membrane,
      20 = optionally, "covering membrane".

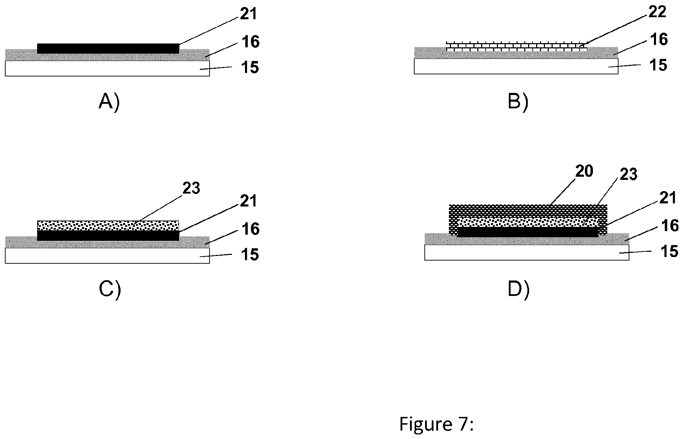

**Figure 7** shows cross-sectional views of an exemplary reference electrode
   A) without "modification"
      15 = substrate,
      16 = insulating layer,
      21 = an electrode,
   B) with Ag/AgCl electrode
      15 = substrate,
      16 = insulating layer,
      22 = an Ag (or Ag/AgCl) electrode,
   C) with "stabilizing layer"
      15 = substrate,
      16 = insulating layer,
      21 = an electrode,
      23 = "stabilizing layer",
   D) with "stabilizing layer" and "covering membrane"
      15 = substrate,
      16 = insulating layer,
      21 = an electrode,
      20 = "covering membrane",
      23 = "stabilizing layer".
**Figure 8** shows an exemplary embodiment for the fabrication of an exemplary single-use monoanalytical test-strip with an additional covering layer. In such exemplary fabrication method, the following steps are performed:
   step 1) provide substrate with insulating layer on top,
   step 2) apply electrode assembly and electrical leads,
   step 3) form analyte-selective electrode and prepare reference electrode,
   step 4) preferably, apply a suitable protective layer, e.g. a plastic insulating material with openings for the electrodes.
      2a = test-strip with protective layer,
      5 = electrode array,
      6 = interface for electrical connection,
      5a = electrode assembly and electrical leads,
      15a = substrate with insulating layer,
      24 = analyte-selective membrane solution and optionally, covering membrane solution for analyte-selective electrode and optionally, stabilizing and/or covering membrane for reference electrode,
      25 = protective layer with openings for electrodes.

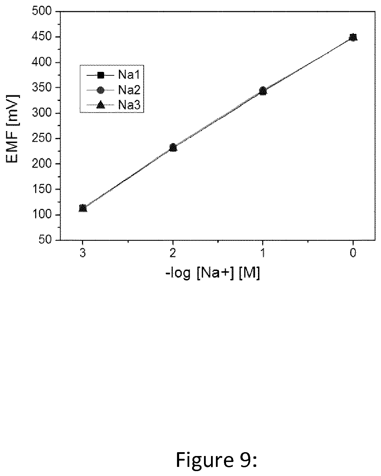

**Figure 9** shows the potentiometric response of fabricated sodium-selective test-strips (Na1-Na3) according to the present invention to different concentrations of sodium in aqueous solutions.

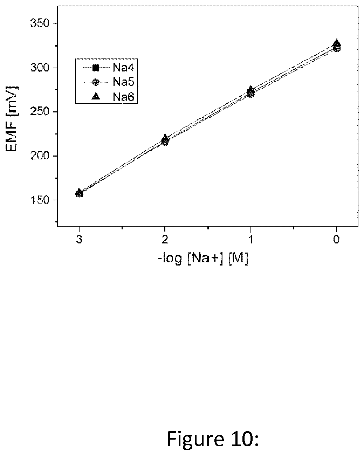

**Figure 10** shows the near-Nernst potentiometric response of exemplary fabricated sodium-selective test-strips (Na4-Na6) according to embodiments of the present invention to different concentrations of sodium in aqueous solutions saturated with a 0.5 M calcium chloride.

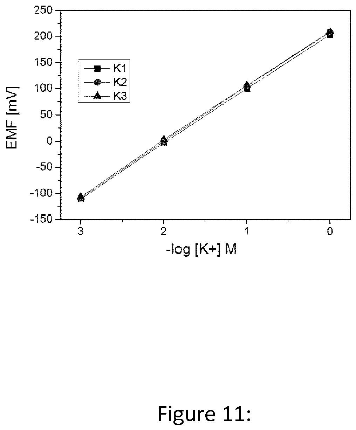

**Figure 11** shows the potentiometric response of fabricated potassium-selective test-strips (K1-K3) according to the present invention to different concentrations of potassium in aqueous solutions.

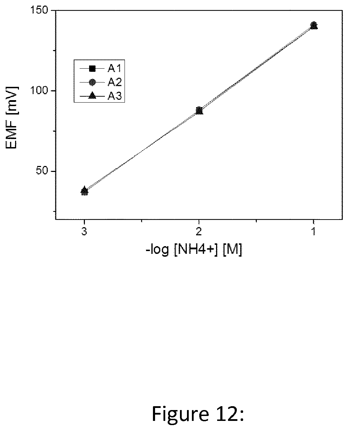

**Figure 12** shows the potentiometric response of fabricated ammonium-selective test-strips (A1- A3) according to the present invention to different concentrations of ammonium in aqueous solutions.

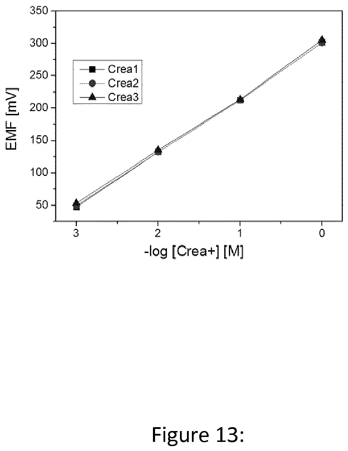

**Figure 13** shows the potentiometric response of fabricated creatinine-selective test-strips (Crea1-Crea3) according to the present invention to different concentrations of creatinine in aqueous solutions.

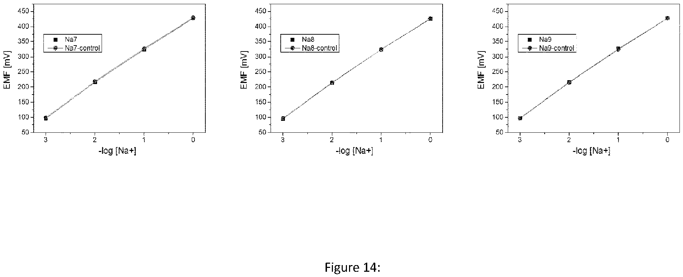

**Figure 14** shows the potentiometric response of fabricated sodium-selective test-strips (Na7-Na9) and reliability by control measurement according to the present invention to different concentrations of sodium in aqueous solutions.

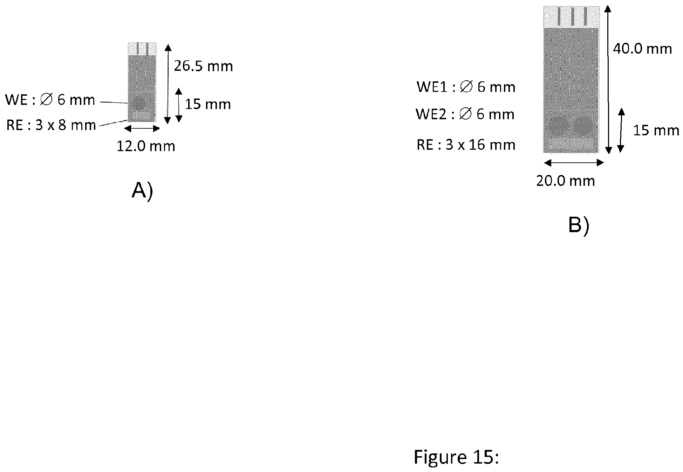

**Figure 15** shows top view of exemplary fabricated single-use monoanalytical test-strips in accordance with embodiments of the present invention with exemplary dimensions of the electrode array and substrate:
   A) comprising WE = working electrode; RE = reference electrode;
   B) comprising WE1 = a first working electrode; RE = reference electrode and WE2 = a second analyte -selective control working electrode or neutral electrode.
Furthermore, the present invention is now further described by reference to the following examples which are given to illustrate, not to limit the present invention.

### EXAMPLES

### Example 1

### Structure of an analyte-selective single-use monoanalytical test-strip

An exemplary fabricated single-use monoanalytical test-strip according to the present invention exhibits exemplary dimensions of the electrode array and substrate as shown in Figure 15. Exemplary patterns of an electrode array and electrical leads applied on an insulating layer are 1) a round shape of a working electrode and a rectangular shape of a reference electrode, 2) a square shape of a working electrode and a rectangular shape of a reference electrode, 3) a round shape of a working electrode and a round shape of a reference electrode, 4) a square shape of a working electrode and a round shape of a reference electrode, 5) a round shape of a working electrode, a rectangular reference electrode, and a second round shape control working electrode, wherein a second analyte-selective electrode is used for control measurement or a neutral electrode is used for determining interferences, 6) a square shape of a working electrode, an oval reference electrode, and a second square shape working electrode, 7) a round shape of a working electrode, a rectangular reference electrode, and a second round shape working electrode wherein a second analyte-selective electrode is used for control measurement or a neutral electrode is used for determining interferences, 8) a round shape of a working electrode, a rectangular reference electrode, a second round shape working electrode wherein a second analyte-selective electrode is used for control measurement and a third round shape working electrode wherein a neutral electrode is used for determining interferences (Figure 5). An analyte-selective working electrode, a reference electrode, a second working electrode (control electrode), and a neutral electrode according to the present invention may have, for example, a shape each independently selected from square shape, rectangular shape, round shape, oval shape. In one embodiment, the reference electrode preferably has a larger surface than the working electrode. In one embodiment, the electrode array is preferably selected from A) exemplary round shape of working electrode and rectangular shape of reference electrode, B) exemplary square shape of analyte-selective working electrode and rectangular shape of reference electrode, and E) exemplary round shape of analyte-selective working electrode and rectangular shape of reference electrode and second round shape control working electrode or neutral electrode (as shown in Figure 5 A,B,E).
Contact paths at the end of electrical leads for contacting a readout-meter device or a test-strip holder unit may each independently have, for example, a line shape, a square shape, a rectangular shape, a round shape, or an oval shape.
An exemplary analyte-selective electrode according to the present invention may be without "inner contact layer", with "inner contact layer", and/or with "covering membrane" (Figure 6). An exemplary reference electrode according to the present invention may be without "modification", an Ag/AgCl electrode, with "stabilizing layer", and/or with "stabilizing layer" and "covering membrane" (Figure 7).

### Example 2

### Fabrication of an analyte-selective single-use monoanalytical test-strip

The following steps are performed to fabricate an exemplary single-use monoanalytical test-strip with an additional protective covering layer:
A plastic substrate, e.g. polycarbonate, polybutylene terephthalate (Valox), with an electrode assembly and electrical leads applied by screen printing is provided. The electrode assembly comprises at least one working electrode, e.g. carbon working electrode, and one reference electrode, e.g. Ag/AgCl system. By means of dispensing or drop casting an analyte-selective membrane solution and optionally, a protective layer solution on the top surface of the working electrode, an analyte-selective electrode is formed. By means of dispensing or drop casting a suitable solution on the top surface of the reference electrode a stabilizing and/or protective layer for the reference electrode is formed. A dielectric protective film is applied on to the monoanalytical test-strip, e.g. by means of screen printing an suitable dielectric ink or applying an adhesive insulating film, with openings for the electrodes. The resulting single-use monoanalytical test-strip has an interface for electrical connection to the POC device.

### Example 3)

### Potentiometric measurements with the prepared test-strips

The test-strip was dipped into the sample solution until all electrodes were covered and the potential difference (EMF) between modified WE(s) and RE was measured with a simple digital voltmeter.

### Example 4)

### Fabrication of a sodium-selective test-strip

A 2-electrode system comprising a carbon working electrode (WE) with ∅ = 6 mm, a (Ag/AgCl) reference electrode (RE) with dimension = 3 x 16 mm and electrical leads that were screen printed onto a 20.0 x 40 mm polybutylene terephthalate (Valox) substrate was used.

To realize a sodium-selective electrode (Na-ISE), an ion selective membrane (Na-ISM) solution (20 µL) was casted on the area of the carbon WE. Then, the substrate was dried 20 min at 60°C to remove solvent. The Na-ISM solution consisted of a mixture of 4.0 mg sodium ionophore X (4-tert-butylcalix[4]arenetetraacetic acid tetraethyl ester), 1.0 mg potassium tetrakis(4-chlorophenyl)borate), 133 mg PVC (high molecular weight polyvinylchloride) and 266 mg o-2-nitrophenyl octyl ether in 3 mL tetrahydrofuran.

### Example 5)

### Sodium test-strip calibration by measuring a sodium solution series with predetermined analyte concentrations

Standard solutions with sodium concentrations of 1M, 10⁻¹M, 10⁻²M, 10⁻³M, respectively, were prepared by dissolving sodium chloride (NaCl) in water. The EMF values were recorded and a calibration curve was set up by plotting the EMF values as a function of the minus logarithm of sodium concentrations. Three different test-strips (Na1 - Na3) were fabricated as described in Example 4 and tested. The results are summarized in Table 1.

**Table 1. EMF values for three different Na⁺ concentrations (1.0 - 0.001 M) obtained by measurements with three different test strips (Na1 - Na3)**

| **c(Na+) [M]** | **EMF [mV] Na1** | **EMF [mV] Na2** | **EMF [mV] Na3** |
|---|---|---|---|
| 10⁰ | 449 | 448 | 448 |
| 10⁻¹ | 342 | 345 | 343 |
| 10⁻² | 231 | 233 | 231 |
| 10⁻³ | 113 | 113 | 111 |

The data clearly show good reproducibility and - as shown in Figure 9 - a logarithmic linear (dynamic) range between 0.001 and 1 M sodium solution. Thus, the linear range of the test-strips covers medically relevant concentrations in physiological fluids, as in human urine where the range for Na concentrations may lie between 0.02-0.25 M.

Out of these data for each test-strip a linear regression equation with the corresponding correlation coefficient R, was calculated (Table 2).

**Table 2. Overview of linear regression equations and correlation coefficients obtained for EMF measurements of aqueous solutions with predetermined analyte-concentrations with three different sodium test-strip (Na1 - Na3).**

| **Test-strip** | **linear regression equation** | **R** |
|---|---|---|
| Na1 | y = -111.9 x + 451.6 | 0.99926 |
| Na2 | y = -111.7 x + 452.3 | 0.99826 |
| Na3 | y = -112.3 x + 451.7 | 0.99866 |

Using these regression equations, the concentration of sodium in a sample can be determined from the measured EMF.

### Example 6)

### Sodium test-strip calibration with stabilized reference electrode potential showing near-Nernst response in a biologically relevant range

The potential of a Ag/AgCl reference electrode (RE) depends on the chloride ions concentration in the sample. For achieving a stable reference electrode potential, a saturating concentration of chloride ions are added to the solutions from which the sensor is calibrated. Therefore, sodium standard solutions with concentrations of 1M, 10⁻¹M, 10⁻²M, 10⁻³M, respectively, were prepared by dissolving sodium chloride (NaCl) in a 0.5 M calcium chloride (CaCl₂) aqueous solution. The sodium sensor was fabricated as described in Example 4 and the EMF values were recorded as described in Example 3. A calibration curve was set up by plotting the EMF values as a function of the minus logarithm of sodium concentrations. Three different test-strips (Na4 - Na6) were fabricated and tested. The results are summarized in Table 3.

**Table 3. EMF values for three different Na⁺ concentrations (1.0 - 0.001 M) obtained by measurements with three different test strips (Na4 - Na6)**

| **c(Na+) [M]** | **EMF [mV] Na4** | **EMF [mV] Na5** | **EMF [mV] Na6** |
|---|---|---|---|
| 10⁰ | 324 | 322 | 328 |
| 10⁻¹ | 272 | 270 | 275 |
| 10⁻² | 217 | 216 | 220 |
| 10⁻³ | 157 | 158 | 159 |

The data clearly show good reproducibility and - as shown in Figure 10 - a logarithmic linear (dynamic) range between 0.001 and 1 M sodium solution.

Out of these data for each test-strip a linear regression equation with the corresponding correlation coefficient R, was calculated (Table 4). The test-strips show a near-Nernst response in the biologically relevant range.

**Table 4. Overview of linear regression equations and correlation coefficients obtained for EMF measurements of 0.5 M CaCl₂ aqueous solutions with predetermined analyte-concentrations with three different sodium test-strip (Na4 - Na6) tested**

| **Test-strip** | **linear regression equation** | **R** |
|---|---|---|
| Na4 | y = -55.6 x + 325.9 | 0.99843 |
| Na5 | y = -54.6 x + 323.4 | 0.99907 |
| Na6 | y = -56.2 x + 329.8 | 0.99841 |

Using these regression equations, the concentration of sodium in a sample can be determined from the measured EMF.

### Example 7)

### Fabrication of a potassium-selective test-strip

A similar 2-electrode system as described in Example 3 was used.
To realize a potassium-selective electrode (Na-ISE), an ion selective membrane (K-ISM) solution (20 µL) was casted on the area of the carbon WE. Then, the substrate was dried 20 min at 60°C to remove solvent. The K-ISM solution consisted of a mixture of 2.0 mg potassium ionophore valinomycin, 0.5 mg potassium tetrakis(4-chlorophenyl)borate, 32.8 mg PVC (high molecular weight polyvinylchloride) and 64.7 mg bis(2-ethylhexyl) sebacate in 1 mL tetrahydrofuran.

### Example 8)

### Potassium test-strip calibration by measuring a potassium solution series with predetermined analyte concentrations

Standard solutions with potassium concentrations of 1M, 10⁻¹M, 10⁻²M, 10⁻³M, respectively, were prepared by dissolving sodium chloride (KCl) in water. The EMF values were recorded and a calibration curve was set up by plotting the EMF values as a function of the minus logarithm of sodium concentrations. Three different test-strips (K1 - K3) were fabricated as described in Example 7 and tested as described in Example 3. The results are summarized in Table 5.

**Table 5. EMF values for three different K⁺ concentrations (1.0 - 0.001 M) obtained by measurements with three different test strips (K1 - K3)**

| **c(K+) [M]** | **EMF [mV] K1** | **EMF [mV] K2** | **EMF [mV] K3** |
|---|---|---|---|
| 10⁰ | 203 | 208 | 209 |
| 10⁻¹ | 100 | 105 | 106 |
| 10⁻² | -3 | 0 | 3 |
| 10⁻³ | -111 | -109 | -107 |

The data clearly show good reproducibility and - as shown in Figure 11 - a logarithmic linear (dynamic) range between 0.001 and 1 M potassium solution. Thus, the linear range of the test-strips covers medically relevant concentrations in physiological fluids, as in human urine where the range for K concentrations may lie between 0.01-0.30 M.

Out of these data for each test-strip a linear regression equation with the corresponding correlation coefficient R, was calculated (Table 6).

**Table 6. Overview of linear regression equations and correlation coefficients obtained for EMF measurements of aqueous solutions with predetermined analyte-concentrations with three different potassium test-strip (K1 - K3).**

| **Test-strip** | **linear regression equation** | **R** |
|---|---|---|
| K1 | y = -104.5 x + 204.0 | 0.99979 |
| K2 | y = -105.6 x + 209.4 | 0.99975 |
| K3 | y = -105.1 x + 210.4 | 0.99960 |

Using these regression equations, the concentration of potassium in a sample can be determined from the measured EMF.

### Example 9)

### Fabrication of an ammonium-selective test-strip

Similar 2-electrode system as described in Example 3 was used.
To realize an ammonium-selective electrode (NH₄-ISE), an ion selective membrane (NH₄-ISM) solution (20 µL) was casted on the area of the carbon WE. Then, the substrate was dried 20 min at 60°C to remove solvent. The NH₄-ISM solution consisted of a mixture of 2.0 mg ammonium ionophore nonactin, 0.5 mg potassium tetrakis(4-chlorophenyl)borate, 32.8 mg PVC (high molecular weight polyvinylchloride) and 64.7 mg 2-nitrophenyl octyl ether in 1 mL tetrahydrofuran.

### Example 10)

### Ammonium test-strip calibration by measuring an ammonium solution series with predetermined analyte concentrations

Standard solutions with ammonium concentrations of 10⁻¹M, 10⁻²M, 10⁻³M, respectively, were prepared by dissolving ammonium sulfate (NH₄)₂SO₄ in water. The EMF values were recorded and a calibration curve was set up by plotting the EMF values as a function of the minus logarithm of sodium concentrations. Three different test-strips (A1 - A3) were fabricated as described in Example 9 and tested as described in Example 3. The results are summarized in Table 7.

**Table 7. EMF values for three different NH₄⁺ concentrations (0.1 - 0.001 M) obtained by measurements with three different test strips (A1 - A3)**

| **c(NH₄⁺) [M]** | **EMF [mV] A1** | **EMF [mV] A2** | **EMF [mV] A3** |
|---|---|---|---|
| 10⁻¹ | 140 | 141 | 140 |
| 10⁻² | 88 | 88 | 87 |
| 10⁻³ | 37 | 37 | 38 |

The data clearly show good reproducibility and - as shown in Figure 12 - a logarithmic linear (dynamic) range between 0.001 and 0.1 M ammonium solution. Thus, the linear range of the test-strips covers medically relevant concentrations in physiological fluids, as in human urine where the range for ammonium concentrations may lie between 0.001-0.1 M.

Out of these data for each test-strip a linear regression equation with the corresponding correlation coefficient R, was calculated (Table 8).

**Table 8. Overview of linear regression equations and correlation coefficients obtained for EMF measurements of aqueous solutions with predetermined analyte-concentrations with three different ammonium test-strip (A1 - A3).**

| **Test-strip** | **linear regression equation** | **R** |
|---|---|---|
| A1 | y = -51.5 x + 191.3 | 0.99715 |
| A2 | y = -52.0 x + 192.7 | 0.99975 |
| A3 | y = -51.0 x + 190.3 | 0.99898 |

Using these regression equations, the concentration of ammonium in a sample can be determined from the measured EMF.

### Example 11)

### Fabrication of a creatinine-selective test-strip

### A similar 2-electrode system as described in Example 3 was used.

To realize a creatinine-selective electrode (Crea-ISE), an ion selective membrane (Crea-ISM) solution (20 µL) was casted on the area of the carbon WE. Then, the substrate was dried 20 min at 60°C to remove solvent. The Crea-ISM solution consisted of a mixture of 1.8 mg creatinium ionophore dibenzo-30-crown-10, 1.0 mg potassium tetrakis(4-chlorophenyl)borate, 32.8 mg PVC (high molecular weight polyvinylchloride) and 64.7 mg 2-nitrophenyl octyl ether in 1 mL tetrahydrofuran.

### Example 12)

### Creatinine test-strip calibration by measuring a creatinine solution series with predetermined analyte concentrations

Standard solutions with calcium concentrations of 1M, 10⁻¹M, 10⁻²M, 10⁻³M, respectively, were prepared by dissolving creatinine hydrochloride in water. The EMF values were recorded and a calibration curve was set up by plotting the EMF values as a function of the minus logarithm of creatinine concentrations. Three different test-strips (Creal - Crea3) were fabricated as described in Example 11 and tested as described in Example 3. The results are summarized in Table 9.

**Table 9. EMF values for three different Crea concentrations (1.0 - 0.001 M) obtained by measurements with three different test strips (Creal - Crea3).**

| **c(Crea⁺) [M]** | **EMF [mV]** | **EMF [mV]** | **EMF [mV]** |
|---|---|---|---|
| | **Crea1** | **Crea2** | **Crea3** |
| 10⁰ | 301 | 301 | 305 |
| 10⁻¹ | 212 | 212 | 213 |
| 10⁻² | 132 | 132 | 135 |
| 10⁻³ | 47 | 49 | 53 |

The data clearly show good reproducibility and - as shown in Figure 13 - a logarithmic linear (dynamic) range between 0.001 and 1 M creatinine solution. Thus, the linear range of the test-strips covers medically relevant concentrations in physiological fluids, as in human urine where the range for creatinine concentrations lies 0.004-0.02 M.

Out of these data for each test-strip a linear regression equation with the corresponding correlation coefficient R, was calculated (Table 10).

**Table 10. Overview of linear regression equations and correlation coefficients obtained for EMF measurements of aqueous solutions with predetermined analyte-concentrations with three different creatinine test-strip (Creal - Crea3).**

| **Test-strip** | **linear regression equation** | **R** |
|---|---|---|
| Crea1 | y = -84.2 x + 299.3 | 0.99942 |
| Crea2 | y = -83.6 x + 298.9 | 0.99930 |
| Crea3 | y = -83.4 x + 301.6 | 0.99823 |

Using these regression equations, the concentration of calcium in a sample can be determined from the measured EMF.

### Example 13)

### Fabrication of a sodium-selective test-strip having a reference electrode with stabilizing layer

A similar 2-electrode system as described in Example 3 was used.

To realize a sodium-selective electrode (Na-ISE), an ion selective membrane (Na-ISM) solution (20 µL) was casted on the area of the carbon WE. On the area of the Ag/AgCl reference electrode, a solution (30 µL) consisting of 78 mg polyvinylbutyral in 1 mL KCl-saturated methanol was casted. Then, the substrate was dried for 20 min at 60°C to remove solvents. The Na-ISM solution consisted of a mixture of 4.0 mg sodium ionophore X (4-tert-butylcalix[4]arenetetraacetic acid tetraethyl ester), 1.0 mg potassium tetrakis(4-chlorophenyl)borate), 133 mg PVC (high molecular weight polyvinylchloride) and 266 mg o-2-nitrophenyl octyl ether in 3 mL tetrahydrofuran.

### Example 14)

### Fabrication of a sodium-selective test-strip comprising a second sodium-selective electrode for control measurement

A 3-electrode system comprising two carbon working electrode (WE1 and WE2) both with ∅ = 6 mm, a (Ag/AgCl) reference electrode (RE) with dimension = 3 x 16 mm and electrical leads that were screen printed onto a 20.0 x 40 mm polybutylene terephthalate (Valox) substrate was used.

To realize a sodium-selective electrode (Na-ISE), an ion selective membrane (Na-ISM) solution (20 µL) was casted on the area of the carbon WE1. To realize a second sodium-selective electrode for control measurement, in similar manner, an ion selective membrane (Na-ISM) solution (20 µL) was casted on the area of the carbon WE2. Then, the substrate was dried for 20 min at 60°C to remove solvent. In both, the Na-ISM solution consisted of a mixture of 3.0 mg sodium ionophore X (4-tert-butylcalix[4]arenetetraacetic acid tetraethyl ester), 1.0 mg potassium tetrakis(4-chlorophenyl)borate), 133 mg PVC (high molecular weight polyvinylchloride) and 266 mg o-2-nitrophenyl octyl ether in 3 mL tetrahydrofuran.

### Example 15)

### Sodium test-strip calibration by measuring a sodium solution series with predetermined analyte concentrations and control measurement with a second sodium-selective electrode

Standard solutions with sodium concentrations of 1M, 10⁻¹M, 10⁻²M, 10⁻³M, respectively, were prepared by dissolving sodium chloride (NaCl) in water. The EMF values between WE1 and RE (EMF1), and between WE2 and RE (EMF2) were recorded. A calibration curve was set up for both by plotting the EMF values as a function of the minus logarithm of sodium concentrations and for control compared. Three different test-strips (Na7 - Na9) were fabricated as described in Example 15 and tested. The results are summarized in Table 11.

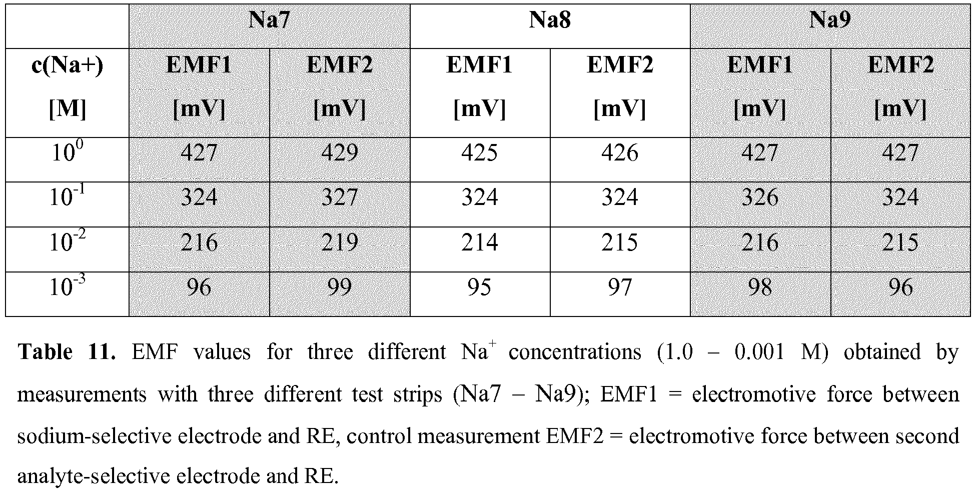

The data clearly show a logarithmic linear (dynamic) range between 0.001 and 1 M sodium solution for all test-strips and - as shown in Figure 14 - a good reproducibility and agreement between the sensor (EMF1) and control measurement (EMF2).

Out of these data for each test-strip a linear regression equation with the corresponding correlation coefficient R, was calculated (Table 12).

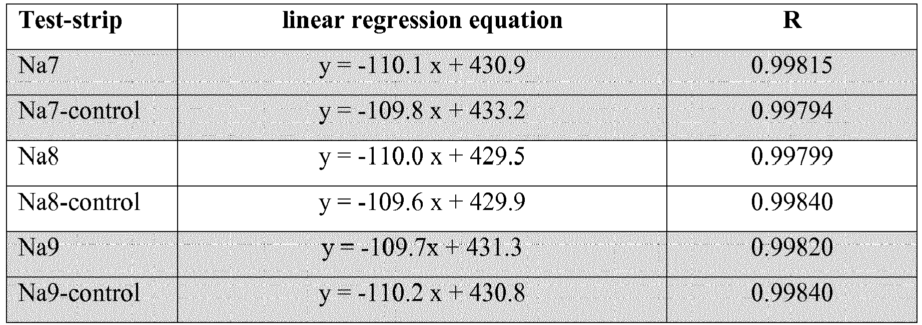

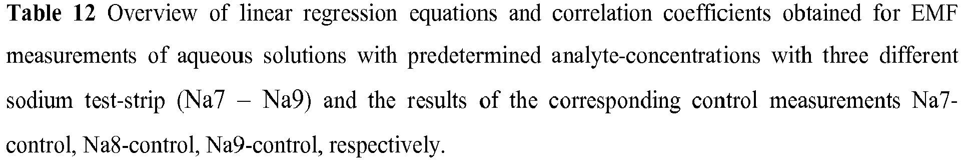

Mean values or values with the best R values can be used for subsequent calculations. By comparing the values and regression equations of the sensor and corresponding control, the high reliability and accuracy of the measurement is shown. The present invention thus allows for measurements of a concentration of an analyte with high accuracy, high reproducibility and high reliability.
Using the regression equations, or the mean values, the concentration of an analyte in a sample can be determined from the measured EMF.

### Proof of function for analyte-specific test-strip

Initial proof of function of analytic-specific test membranes loaded on fabricated plastic test-strips with screen printed electrodes was obtained for various analytes, including sodium, potassium, ammonium, calcium, creatinine by
- successful potentiometric measurements of concentrations of an analyte in fluids with predetermined concentrations of an analyte
- proven linear (dynamic) range of the test-strips for relevant concentrations of an analyte in physiological fluids
- proven near-Nernst potentiometric response for relevant concentrations of sodium in relevant concentrations of physiological fluids
- proven reliable potentiometric response for relevant concentrations of sodium, potassium, ammonium, and creatinine in relevant concentrations of physiological fluids

Consequently, a single-use monoanalytical test-strip and a non-invasive point-of-care (POC) device according the present invention, as well as a method for quantitatively determining the concentration of an analyte and a method for detecting deficiency and/or overload of an analyte in patient's body according to the present invention, are easy-to-use, non-invasive means to identify and monitor concentrations of an analyte in a body.

### REFERENCES

[1] Fluids, Electrolytes and Acid-Base Disorders Handbook, Editors: Canada TW, Tajchman SK, Tucker AM, Ybarra JV. ASPEN (2015); eBook edition: ISBN-13: 978-1-889622-18-7
[2] Acid-Base and Electrolyte Handbook for Veterinary Technicians, Editors: Randels-Thorp A, Liss D. Wiley (2017)
[3] Braun MM, Barstow CH, Pyzocha NJ. Diagnosis and Management of Sodium Disorders: Hyponatremia and Hypernatremia. Am Fam Physician. 91(5); 299-307 (2015)
[4] Moritz ML, Ayus JC. Disorders of water metabolism in children: hyponatremia and hypernatremia. Pediatr Rev 23; 371-380 (2002)
[5] Moritz ML, Ayus JC. Maintenance intravenous fluids in acutely ill patients. N Engl J Med 373; 1350-60 (2015)
[6] Trieu K, Neal B, Hawkes C, Dunford E, Campbell N, Rodriguez-Fernandez R, et al. Salt Reduction Initiatives around the World - A Systematic Review of Progress towards the Global Target. PLoS One. 10(7): e0130247 (2015)
[7] Subasinghe AK, Arabshani S, Businye D, Evans RG, Walker KZ, Ridell MA, Thrift AG. Association between salt and hypertension in rural and urban populations of low and middle income countries: a systematic review and metaanalysis of population-based studies. Asia Pac J Clin Nutr 25:402-13 (2016)
[8] Upadhyay A, Jaber BL, Madias NE. Incidence and prevalence of hyponatremia. Am J Med 1; S30-S35 (2006)
[9] Blank MC, Bedarf JR, Russ M, Grosch-Ott S, Thiele S, Unger JK. Total body Na(+)-depletion without hyponatremia can trigger overtraining-like symptoms with sleeping disorders and increasing blood pressure: explorative case and literature study. Med Hypotheses 79; 799-804 (2012)
[10] Heinz-Erian P, Akdar Z, Haerter B, Waldegger S, Giner T, Scholl-Buergi S, Müller T. Decreased urinary-sodium-to-creatinine ratio identifies sodium depletion in pediatric acute gastroenteritis. Klin Padiatr 228; 24-8 (2016)
[11] Rodriguez MJ, Alcaraz A, Solana MJ et al. Neurological symptoms in hospitalized patients: do we assess hyponatremia with sufficient care? Acta Paediatr 103; e7-e10 (2014)
[12] Corona G, Guiliani C, Parenti G et al. Moderate hyponatremia is associated with increased risk of mortality: evidence from a meta-analysis. PLOS ONE 8; e80451 (2013)
[13] Viera AJ, Wouk N. Potassium Disorders: Hypokalemia and Hyperkalemia. Am Fam Physician. 92(6):487-495 (2015)
[14] Heath D, Marx SJ. Clinical Endocrinology 2: Calcium Disorders, A volume in Butterworths Medical Reviews (1982)
[15] Moe SM. Disorders Involving Calcium, Phosphorus, and Magnesium. Prim Care 35(2); 215-234 (2008)
[16] Saris NL, Mervaala E, Karppanen H, Khawaja JA, Lewenstam A. Magnesium, an update on physiological, clinical and analytical aspects. Clin. Chim. Acta 294; 1-26 (2000)
[17] Kamel KS, Ethier JH, Richardson RMA, Bear RA, Halperin M. Urine electrolytes and osmolality: when and how to use them. Am J Nephrol 10; 89-102 (1990)
[18] Manghat P, Sodi R, Swaminathan R. Phosphate homeostasis and disorders. Annals of Clinical Biochemistry 51(6); 631-656 (2014)
[19] Patrick L, Iodine: Deficiency and therapeutic considerations, Alt Med Rev. 13; 116-127 (2008)
[20] Ackland ML, Michalczyk A. Zinc deficiency and its inherited disorders -a review. Genes Nutr 1(1); 41-50 (2006)
[21] Romana DL, Olivares M, Uauy R, Araya M. Risks and benefits of copper in light of new insights of copper homeostasis. J Trace Elem Med Biol 25; 3-13 (2011)
[22] Licht RW. Lithium: still a major option in the management of bipolar disorder. CNS Neurosci Ther. 18(3); 219-226 (2012)
[23] de Burbane C, Buchet JP, Leroyer A, et al. Renal and neurologic effects of cadmium, lead, mercury, and arsenic in children: evidence of early effects and multiple interactions at environmental exposure levels. Environ Health Perspect 114; 584-590 (2006)
[24] Martin MD, Woods JS, Leroux BG, Rue T, Derouen TA, Leitao J, et al. Longitudinal urinary creatinine excretion values among preadolescents and adolescents. Transl Res Jan 151(1); 51-6 (2008)
[25] Newman DJ, Price CP. Renal function and nitrogen metabolites. In Tietz Textbook of Clinical Chemistry. Third Edition. Edited by Burtis CA, Ashwood ER. pp 1204-1270 (1990)
[26] Puche RC, Vacarro D, Sanchez A, et al. Increased fractional excretion of sulphate in stone formers. Br J Urol 71; 523-526 (1993)
[27] Bühlmann P, Pretsch E, Bakker E. Carrier-Based Ion-Selective Electrodes and Bulk Optodes. 2. Ionophores for Potentiometric and Optical Sensors. Chem. Rev. 98; 1593-1687 (1998)
[28] Vashist SK, Luppa PB, Yeo L Y, Ozcan A, Luong JHT, Emerging Technologies for Next-Generation Point-of-Care Testing. Trends in Biotechnology, 33, 11; 692-705 (2015)
[29] John AS, Price CP. Existing and Emerging Technologies for Point-of-Care Testing. Clin Biochem Rev 35(3); 155-167 (2014)

The features of the present invention disclosed in the specification, the claims, and/or in the accompanying figures may, both separately and in any combination thereof, be material for realizing the invention in various forms thereof.

## Claims

1. A single-use monoanalytical test-strip for the quantitative determination of an analyte in a patient's urine sample, wherein said analyte is selected from cations of alkali metals, preferably selected from Na⁺, K⁺, and Li⁺, alkaline earth metals, preferably selected from Ca²⁺ and Mg²⁺, other metals, which are non-alkali metals and non-alkaline earth metals, preferably selected from Pb²⁺, Zn²⁺, and Cu²⁺, anions selected from halides, in particular Cl⁻ and I⁻, phosphates, and sulfates, and non-metal cations, preferably selected from NH₄⁺, H⁺, and creatinine⁺,
said test-strip comprising:
- a substrate which either is electrically insulating or which has an electrically insulating layer applied thereon,
- an electrode assembly applied on said substrate or on said electrically insulating layer, if present; said electrode assembly comprising at least
- one analyte-selective working electrode;
- one reference electrode for said analyte-selective working electrode;
- optionally; a second analyte-selective working electrode for control measurement, and/or one or two neutral electrodes for measuring and eliminating interferences,
- an interface for electrically connecting said electrode assembly to a read out-meter device, optionally via a test-strip holder unit.

2. The single-use monoanalytical test-strip according to claim 1, wherein said analyte-selective working electrode, said reference electrode, and said second analyte-selective electrode and/or neutral electrode(s), if present, have been applied on said substrate or on said electrically insulating layer, if present, by a suitable deposition technique, such as printing, sputtering, evaporating, electro-less plating, affixation, gluing or lithography, preferably screen printing or ink-jet printing, thus forming an electrode assembly on said substrate or on said electrically insulating layer, and wherein said analyte-selective working electrode comprises an analyte-selective membrane, and wherein said neutral electrode(s) comprise(s) a membrane that is not analyte-selective.

3. The single-use monoanalytical test-strip according to any of the foregoing claims, wherein said substrate is made of a material selected from plastic, ceramic, alumina, paper, cardboard, rubber, textile, carbon-based polymers, such as polypropylene, fluoropolymers, such as Teflon, silicon-based substrates, such as glass, quartz, silicon nitride, silicon oxide, silicon based polymers such as polydimethoxysiloxane, semiconducting materials such as elemental silicon, dielectric materials, preferably selected from organic dielectric materials such as polyimide, polycarbonate, polyvinyl chloride, polystyrene, polyethylene, polypropylene, polyester, polyethylene terephthalate, polyurethane, polyvinylidene fluoride, inorganic dielectric materials such as silicium dioxide, and wherein said electrically insulating layer, if present, is made of a dielectric material, wherein, if said electrically insulating layer is present on said substrate, said electrode assembly is located on said electrically insulating layer.

4. The single-use monoanalytical test-strip according to any of the foregoing claims, wherein said analyte-selective working electrode comprises an analyte-selective membrane that comprises an analyte-selective carrier in a polymer matrix.

5. The single-use monoanalytical test-strip according to any of the foregoing claims, wherein said electrode assembly further comprises one or two neutral electrodes for measuring and eliminating interferences, wherein said neutral electrode(s) comprise(s) a membrane comprising a polymeric matrix without any analyte-selective carrier.

6. The single-use monoanalytical test-strip according to any of the foregoing claims, wherein said electrode assembly further comprises a second analyte-selective working electrode for control measurement.

7. The single-use monoanalytical test-strip according to any of the foregoing claims, wherein each of said electrodes in said electrode assembly has an electrical lead, respectively, wherein said electrical lead connects said electrode with said interface for electrically connecting said electrode assembly to a readout-meter device.

8. The single-use monoanalytical test-strip according to any of the foregoing claims, wherein said reference electrode has a surface larger than the surface of said working electrode.

9. The single-use monoanalytical test-strip according to any of the foregoing claims, wherein said test-strip is a dipstick test-strip which is contacted with a sample by dipping.

10. A non-invasive point-of-care (POC) device for detecting and/or determining deficiency and/or overload of an analyte in a patient's body, said POC device comprising:
a readout-meter device for the quantitative and selective measurement of the concentration of an analyte in a urine sample, said readout-meter device comprising
- a multichannel amplifier, preferably having high input resistance, for amplifying electrical signal(s) transmitted from a single-use test-strip
- a controller including an analog/digital converter and a storage memory, for converting electrical signals received from a single-use test-strip into analyte concentration measurement(s)
- a measurement mechanism to start and, preferably, conduct a measurement of an analyte concentration
- an output device for indicating concentration measurements to a user, preferably a display
- a receiving module for receiving an interface of a single-use test-strip or of a test-strip holder unit,
- optionally, a communication unit, and
- a power supply; and
optionally, a test-strip holder unit for receiving an interface of a single-use test-strip and having an interface for connecting to said receiving module and for establishing electrical contact between said readout-meter device and an electrode assembly of said single-use test-strip, thus allowing the detection and transmission of electrical signal(s) from said single-use test-strip to said readout-meter device, wherein said test-strip holder unit has electrical connectors for separately contacting each electrode via said interface of said test-strip.

11. A non-invasive point-of-care (POC) device according to claim 10, said POC device further comprising
a release mechanism to release said test-strip from said receiving module of the readout-meter device or from said test-strip holder unit.

12. The non-invasive point-of-care (POC) device according to claim 10 or 11, wherein said POC device comprises a test-strip holder unit.

13. The non-invasive point-of-care (POC) device according to any of claims 10 - 12, wherein said measurement mechanism is selected from a measure button, such as a push button, a press button, a touch button, or a digital control button, a lever, a second pressure point of a button of the POC device or pressing a button of the POC device for a second time, and a voice-controlled means to initiate the measurement.

14. The non-invasive point-of-care (POC) device according to any of claims 11 - 13, wherein said release mechanism is selected from a button, such as a push button, a press button, a touch button, or a digital control button, a lever, a second pressure point of a button of the POC device or pressing a button of the POC device for a second time, and a voice-controlled release mechanism.

15. The non-invasive point-of-care (POC) device according to claim 11-14, wherein said release mechanism is part of the readout-meter device, or wherein said release mechanism is part of the test-strip holder unit.

16. The non-invasive point-of-care (POC) device according to any of claims 10-15, wherein said device further comprises a user-interface for operating said device, and/or a memory for storing a plurality of analyte concentration measurements, and/or a communication unit, preferably a USB and/or wireless communication unit, for transferring and/or exchanging data with an external computer or external network.

17. The non-invasive point-of-care (POC) device according to any of claims 10-16, further comprising a single-use test-strip inserted into said receiving module of said readout-meter device or said test-strip holder unit by way of said interface of said single-use test-strip, thus establishing electrical contact between said electrode assembly of said test-strip and said readout-meter device, wherein, preferably, said single-use test-strip is the single-use monoanalytical test-strip according to any of claims 1-9.

18. A method for quantitatively determining the concentration of an analyte in a patient's urine sample, comprising the steps:
a) providing a urine sample
b) connecting a single-use test-strip comprising an electrode assembly, wherein said electrode assembly comprises at least
- one analyte-selective working electrode;
- one reference electrode for said analyte-selective working electrode;
- optionally; a second analyte-selective working electrode for control measurement, and/or one or two neutral electrodes for measuring and eliminating interferences,
to a readout-meter device of a point-of-care (POC) device as defined in any of claims 10-16, to assemble a point-of-care (POC) device as defined in claim 17, wherein said single-use test-strip is inserted into said receiving module of said readout-meter device or into said test-strip holder unit contacted to said readout meter device, thus establishing electrical contact between said electrode assembly of said test-strip and said readout-meter device,
c) contacting said test-strip with said urine sample, and allowing said electrode assembly of said test-strip to be wetted by and come into contact with said urine sample, optionally withdrawing the urine-wetted test-strip from said urine sample,
wherein said connecting of said test-strip to said readout-meter device of said POC device, optionally via said test-strip holder unit, of step b) occurs either before or after step c), d)
measuring the concentration of an analyte in said urine sample, using said point-of-care (POC) device assembled in step b), wherein, preferably, said single-use test-strip is the single-use monoanalytical test-strip of any of claims 1-9.

19. A method of detecting and/or determining deficiency and/or overload of an analyte in a patient's body, said method comprising the steps:
- performing the method according to claim 18
- detecting and/or determining an analyte deficiency, if the detected concentration in said urine sample is lower than a physiologically adequate value for the patient, and detecting and/or determining an analyte overload, if the detected concentration is higher than a physiologically adequate value for the patient.

20. The method according to any of claims 18-19, wherein said analyte is selected from cations of alkali metals, preferably selected from Na⁺, K⁺, and Li⁺, alkaline earth metals, preferably selected from Ca²⁺ and Mg²⁺, other metals, which are non-alkali metals and non-alkaline earth metals, preferably selected from Pb²⁺, Zn²⁺, and Cu²⁺, anions selected from halides, in particular Cl⁻ and I⁻, phosphates, and sulfates, and non-metal cations, preferably selected from NH₄⁺, H⁺, and creatinine⁺.
